(19) Europäisches Patentamt
European Patent Office
Office européen des brevets

(11) EP 2 698 385 A1

(12) EUROPEAN PATENT APPLICATION

(43) Date of publication:
**19.02.2014 Bulletin 2014/08**

(51) Int Cl.:
***C07K 16/44** (2006.01)* ***G01N 33/94** (2006.01)*

(21) Application number: **12180490.0**

(22) Date of filing: **14.08.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Randox Laboratories Ltd.
Crumlin, County Antrim BT29 4QY (GB)**

(72) Inventors:
- **Fitzgerald, Stephen Peter
Crumlin
Antrim BT29 4QY
Northern Ireland (GB)**
- **Innocenzi, Paul John
Crumlin
Antrim BT29 4QY
Northern Ireland (GB)**
- **Lowry, Philip Andrew
Crumlin
Antrim BT29 4QY
Northern Ireland (GB)**
- **McConnell, Ivan Robert
Crumlin
Antrim BT29 4QY
Northern ireland (GB)**
- **Benchikh, Elouard
Crumlin
Antrim BT29 4QY
Northern Ireland (GB)**

(74) Representative: **O'Connell, Maura
FRKelly
27 Clyde Road
Ballsbridge
Dublin 4 (IE)**

## (54) Detection of synthetic cannabinoids

(57) The invention describes methods and kits for detecting and determining current and future synthetic cannabinoids from the JWH and CP families. Unique antibodies derived from immunogens enable said methods and kits.

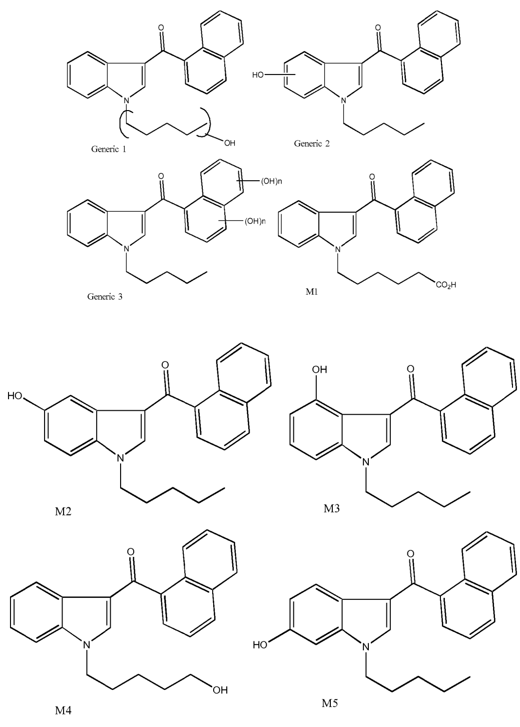
Generic 1 Generic 2
Generic 3 M1
M2 M3
M4 M5

**Figure 7**

EP 2 698 385 A1

## Description

## BACKGROUND

The increasing rise in the use of stealth drugs (novel synthetic drugs that were previously or remain analytically/structurally uncharacterised and unclassified by government institutions), is exemplified by synthetic cannabinoid products which incorporate JWH-018 as the active ingredient. Stealth synthetic cannabinoid (SSC) drug manufacturers can base their choice of active molecular target on scientific literature studies that address the therapeutic potential of $CB_1$ (the CNS cannabinoid receptor) agonists and antagonists. By incorporating novel, analytically uncharacterised compounds with high $CB_1$ receptor affinity into herbal mixtures (packaged under such names as Spice, Yucatan Fire), the manufacturers are able to legally target drug consumers clandestinely by promoting the material as herbal therapeutics. A problem for governments and drug enforcement agencies is that even after identifying and banning a new synthetic cannabinoid, the manufacturers can rapidly react to the banning by incorporating a different active analogue into the same or a different herbal product; targeted minor changes in the molecular structure of the known active compound can preserve receptor activity but often produces a molecule whose GC-MS/LC-MS (the commonly applied detection techniques) profile is completely different from the original active molecule. Hence the new active molecule initially remains unidentified and a further resource intensive and costly chemical analytical study to enable structural characterisation is required. The main active ingredients highlighted in SSC products to date are JWH-018, CP 47,497 and JWH-073 (Uchiyama et al. 2010; Hudson et al. 2010; Dresen et al. 2010). Initial studies of the metabolism of JWH compounds have highlighted metabolic processes similar to tetrahydrocannabinol (THC) metabolism, namely ring and alkyl substituent hydroxylation, carboxylation and glucuronidation. As described herein, unless otherwise stated, JWH refers to molecules comprising structure I which are $CB_1$-active or metabolites of the $CB_1$-active parent, in which the indole ring system is present as a fused heterobicyclic i.e. it is not part of, for example, a fused heterotricyclic ring system. Y can be hydrogen or a substituted or unsubstituted alkyl group such as butyl, pentyl or 2-(morpholin-4-yl)ethyl, while R is a carbon atom which may be part of a fused or unfused, substituted or unsubstituted aromatic ring or a substituted or unsubstituted alkyl, alkenyl or alkynyl chain optionally attached to a fused or unfused, substituted or unsubstituted aromatic ring, but is usually a substituted or unsubstituted naphthyl ring.

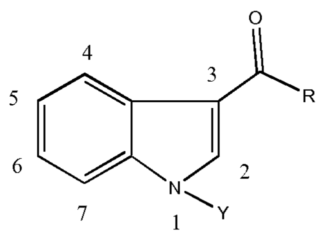

Structure I

Indolyl, naphthyl, carboxyalkyl, N-dealkylated and N-alkyl mono-, di-, and trihydroxylated metabolites, as well as their glucuronidated conjugates are reported JWH-018 metabolites (Sobolevsky et al. 2010; Kraemer et al. 2008). Moller et al. (2010) highlighted the same metabolites as Sobolevsky et al. (2010), with the monohydroxylated N-alkyl chain being the most abundant phase I metabolite; Wintermeyer et al. (2010) conducted an *in vitro* study that largely confirmed previous findings. Herbal therapeutics have been analysed using solvent extraction, pre-derivatisation and finally GC-MS analysis in SIM mode (Rana et al. 2010). This method is inadequate for the detection of future and 'current' JWH SSCs (it is conceivable that 'current' herbal therapeutics, as well as JWH-018, incorporate JWH a SSCs that are not yet characterised), requires sample pre-derivatisation, specialist staff for its implementation and expensive equipment. In order to address the problem associated with the cheap and rapid detection of known JWH molecules and their metabolites and/or future and associated metabolites based on the JWH drug families, the Inventors devised a novel method based on novel antibodies raised from novel immunogens. The antibodies underpin an effective analytical and economic solution to the detection and quantification of current and future JWH $CB_1$-active molecules in *in vitro* patient samples and herbal therapeutics.

## References

Dresen et al. (2010). J. Mass Spectrom., 45: 1186-1194.
Kraemer et al. (2008). http://www.gtfch.org/cms/images/stories/ media/tk/tk76_2/ abstractsvortraege.pdf.

Hudson et al. (2010). J. Anal. Toxicol., 34: 252-260.
Moller et al. (2010). Drug Test Anal. Sep 24. [Epub ahead of print].
Rana et al. (2010). Quantitative composition of synthetic cannabinomimetics in "Herbal High" products. Poster at SOFT 2010 Annual Meeting, Richmond, Virginia, US.
Sobolevsky et al. (2010). Foren. Sci. Int., 200(1-3): 141-147.
Uchiyama et al. (2010). Foren. Sci. Int., 198: 31-38.
Wintermeyer et al. (2010). Anal. Bioanal. Chem., 398: 2141-2153.

## SUMMARY OF THE INVENTION

The invention describes a rapid and practical method for the detection and determination of known and/or stealth synthetic cannabinoids based on the JWH drug family. Kits and their use for JWH SSC detection and determination in herbal therapeutics and *in vitro* patient samples are also described. The invention is underpinned by novel immunogens and antibodies which enable said methods, kits and applications.

## DRAWINGS

Figure 1 Synthesis of Hapten-A. (used to generate Immunogen I)
Figure 2 Synthesis of Hapten-B. (used to generate Immunogen II)
Figure 3 Synthesis of Hapten-C. (used to generate Immunogen VIII)
Figure 4 Synthesis of Hapten-D. (used to generate Immunogen IV)
Figure 5 Immunogens I, II and III. (Immunogen III now renamed as Immunogen VIII)
Figure 6 Representative molecules of the JWH family.
Figure 7 Identified and hypothesised metabolites of JWH (n=1-2). The hydroxylated metabolites are potentially further metabolised to the corresponding glucuronide(s).
Figure 8 $CB_1$-active molecules of the CP family (available from Cayman Chemical company, 1180 East Ellsworth Road, Ann Arbor, Michigan 48108, USA).
Figure 9: Chemical Structures of JWH-081, JWH-018, JWH-122, JWH-210, JWH-182 and JWH-398
Figure 10: Chemical Structures of JWH-015, JWH-016 and JWH-007
Figure-11: Chemical Structure of Hapten-1
Figure-12: Chemical Reactions of the Synthesis of Hapten-1
Figure 13: Tracers used in the invention
Figure 14: Immunogens of the invention

## DETAILED DESCRIPTION OF THE INVENTION

A first aspect of the invention is an antibody raisable against one or more immunogens possessing the following structures

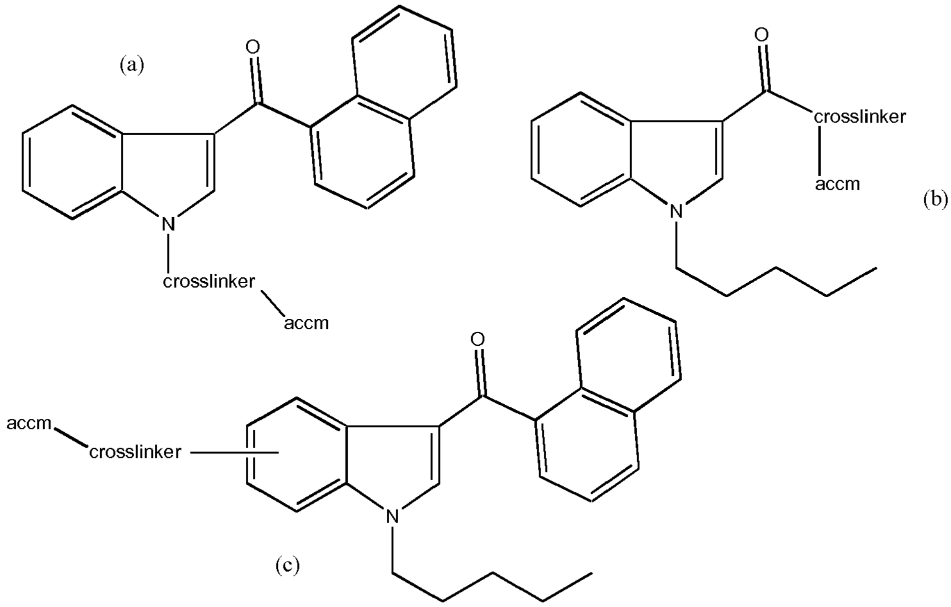

(a)

(b)

(c)

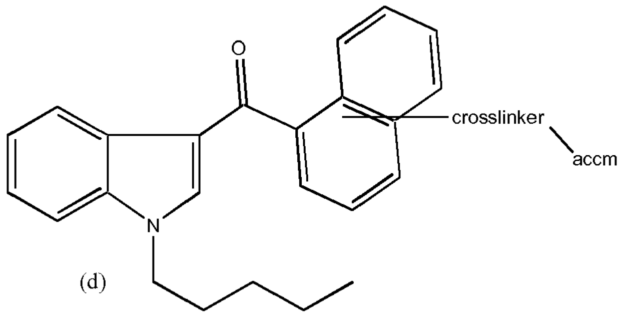

(d)

Group I (a)-(d) (immunogens of the JWH family)

in which the accm is an antigenicity conferring carrier material; the crosslinker is a functionalised linking group joining the accm to the remainder of the molecule and in which, in structure (a), the 2-position of the indole group can optionally be substituted with a methyl group.

The antibody may bind to an epitope of structure

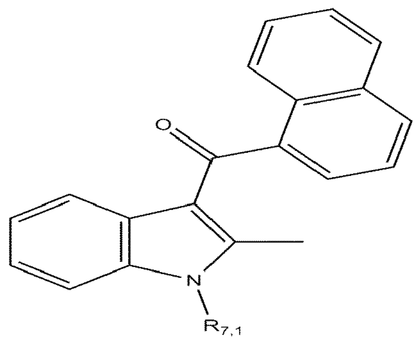

wherein,

$R_{7,1}$ is a $C_3$ or $C_4$ hydrocarbon chain. The antibody may bind to an epitope of the molecules JWH-015 and JWH-016. The antibody may be further characterised by being raisable from an immunogen of structure VII. Alternatively or additionally, the antibody may be further characterised by having a $B/B_0$ of ≤ 20% for all cross-reactants listed in Table 4 as having a $B/B_0$ below the indicated cut-off (standardised with RCS-4 and using tracer ESC6585).

The antibody may bind to an epitope of structure

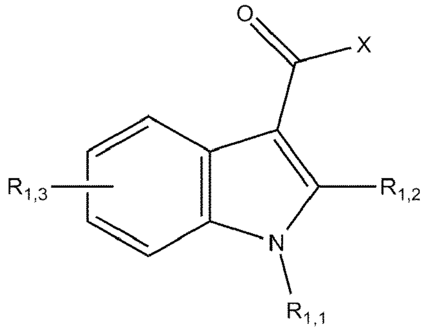

wherein,

$R_{1.1}$ is H or a $C_1$ to $C_6$, substituted or unsubstituted, saturated, hydrocarbon chain;
$R_{1,2}$ is H or methyl, optionally H;
$R_{1,3}$, if present, is methoxy or OH; and
X is a substituted or unsubstituted naphthyl moiety, optionally an unsubstituted naphthyl moiety, or a substituted benzyl moiety. Optionally, $R_{1,1}$ is selected from H; substituted methyl, substituted ethyl, substituted or unsubstituted propyl, substituted or unsubstituted butyl, substituted or unsubstituted pentyl or hexyl; wherein $R_{1,1}$ is, optionally, selected from (1-methyl-2-piperidinyl)methyl, ethyl-morpholino, propyl, substituted propyl selected from 1,2-dimethylpropyl, 2,2-dimethylpropyl or butyryl; butyl, substituted butyl selected from valeryl, 1-methylbutyl, 2-metylbutyl, 3-methylbutyl, 3-hydroxybutyl, 4-hydroxybutyl, pentyl, substituted pentyl selected from 4-hydroxypentyl, 5-hydroxypentyl, 5-fluoropentyl, 4-fluoropentyl and 4-hydroxy-5-fluoropentyl, 4-pentenyl and hexyl. Optionally, $R_{1,3}$, if present, is present at one of positions 5, 6 or 7 of the indole ring, optionally, at one of positions 6 or 7 of the indole ring. Further optionally, when X is substituted naphthyl, the naphthyl is substituted at the 6-position of the naphthyl ring with, optionally, methyl or, when X is substituted phenyl, the phenyl is substituted at position 2 of the phenyl ring with, optionally, iodine. The antibody may be characterised by being raisable from an immunogen of structure I. The antibody may be further characterised by having a $B/B_0$ of ≤ 16% for all cross-reactants listed in Table 4 as having a $B/B_0$ below the indicated cut-off (standardised with JWH-018 and using tracer ESC6557).

The antibody may bind to an epitope of structure

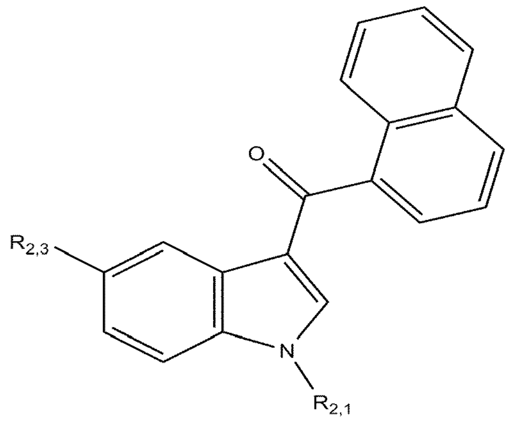

wherein,

$R_{2,1}$ is $C_4$ to $C_6$, substituted or unsubstituted, saturated or unsaturated, hydrocarbon chain; and
$R_{2,3}$ is H or hydroxyl.. Optionally, $R_{2,1}$ is selected from butyl, pentyl, substituted pentyl selected from 4-fluoropentyl and 5-fluoropentyl, 4-pentenyl and hexyl. The antibody may be further characterised by having been derived from an immunogen of structure II. Alternatively or additionally, the antibody further characterised by having a $B/B_0$ of ≤ 15% for all cross-reactants listed in Table 4 as having a $B/B_0$ below the indicated cut-off (standardised with JWH-018 and using tracer ESC6557).

The antibody may bind to an epitope of structure

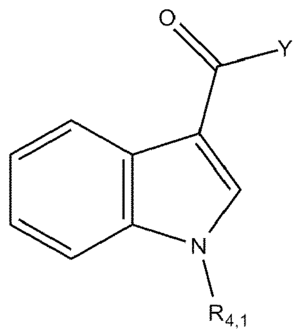

wherein,

$R_{4,1}$ is a $C_4$ or $C_5$, substituted or unsubstituted, saturated or unsaturated, hydrocarbon chain;
Y is substituted or unsubstituted naphthyl moiety or substituted phenyl moiety, in which Y is optionally a substituted naphthyl moiety. Optionally, $R_{4,1}$ is selected from butyl, substituted butyl selected from 3-methylbutyl; pentyl; substituted pentyl selected from 4-fluoropentyl and 5-fluoropentyl; and 4-pentenyl. Optionally, Y is substituted naphthyl and the naphthyl is substituted with methyl, ethyl, propyl, methoxy or chlorine, optionally at one of positions 4, 5, 6 or 7 of the naphthyl ring. Further optionally, Y is substituted phenyl, the phenyl is substituted with methoxy or iodine, optionally at position 2 of the phenyl ring and $R_{4,1}$ is selected from pentyl and 5-fluoropentyl. Further optionally, Y is substituted naphthyl and the naphthyl is substituted with methyl or methoxy at position 4 of the naphthyl ring and $R_{4,1}$ is pentyl. The antibody may be further further characterised by being raisable from an immunogen of structure IV. Alternatively or additionally, the antibody may be characterised by having a $B/B_0$ of $\leq$ 15% for all cross-reactants listed in Table 4 as having a $B/B_0$ below the indicated cut-off (standardised with RCS-8 and using tracer ESC6557).

The antibody may bind to an epitope of structure

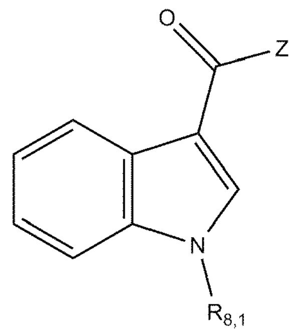

wherein,

$R_{8,1}$ is a $C_4$ or $C_5$, substituted or unsubstituted, hydrocarbon chain;
Z is substituted or unsubstituted 1- or 2-naphthyl, adamantyl, substituted benzyl or substituted phenyl moiety. Optionally, $R_{8,1}$ is selected from butyl; pentyl; and substituted pentyl, optionally selected from 5-fluoropentyl, 4-fluoropentyl and 4-hydroxypentyl. Optionally, when Z is substituted 1- or 2-naphthyl, the naphthyl is substituted with methyl or methoxy, optionally at the 4 position on the naphthyl ring or, when Z is substituted benzyl, the benzyl is substituted with methyl optionally at the ortho or meta positions on the phenyl ring, methoxy or chlorine or, when $R_3$ is substituted phenyl, the phenyl is substituted with methoxy optionally at the meta or para positions of the phenyl ring or iodine. The antibody may be further characterised by being raisable from an immunogen of structure VIII. Alternatively or additionally, the antibody of may be characterised by having a $B/B_0$ of $\leq$ 20% for all cross-reactants listed in Table 4 as having a $B/B_0$ below the indicated cut-off (standardised to JWH-018 and using tracer ESC5794).

By functionalised, it is meant the crosslinker incorporates atoms that enable it to bond to both the accm and the JWH moiety, forming a bridging group. In structure (c) the crosslinker extends from the 4, 5, 6 or 7-position of the indole ring and in structure (d) the crosslinker extends from the 2, 3, 4, 5, 6, 7, or 8-position of the naphthyl ring. The

crosslinker concept is well known to the person skilled in immunogen synthesis. For the current invention, when conjugating the hapten to the accm to form the immunogen, the nature and length of the crosslinker follows standard methods in order to optimise hapten epitopic recognition by the antibody. This entails a crosslinker of low immunogenicity and a chain length preferably of no greater than about ten atoms, most preferably no greater than six atoms.

Preferably for structure (a) the crosslinker is $-(CO)_n-D-Y-$ and where n= 0 or 1, and D is a $C_{1-10}$, preferably a $C_{1-5}$ substituted or unsubstituted straight chain alkylene or arylene moiety and Y, which is attached to the accm, is selected from groups such as carbonyl, amino, thiol, maleimide, isocyanato, isothiocyanato, aldehyde, diazo and dithiopyridyl. Y is preferably carbonyl or amino. Optionally, the 2-position on the indole ring may be substituted with methyl.

Preferably for structures (b), (c) and (d), the crosslinker is $-(A)_n- (D)_p- Y-$ where A= O, -N(R)-, S, -S(O)- (sulphoxide) or $-S(O)_2-$ (sulphonyl) and R=H or $C_{1-5}$ alkyl, n= 0 or 1, p= 0 or 1 and D is a $C_{1-10}$, preferably a $C_{1-5}$ substituted or unsubstituted straight chain alkylene or arylene moiety and Y, which is attached to the accm, is selected from groups such as carbonyl, amino, thiol, maleimide, isocyanato, isothiocyanato, aldehyde, diazo and dithiopyridyl. Y is preferably carbonyl or amino.

Preferred immunogens correspond to structures (a) and (c). The crosslinker of structure (c) of Group I is preferably attached to the 5-indole position. The crosslinker of structure (d) of Group I is preferably attached to the 4-naphthyl position. It has been found that immunogens of the invention raise antibodies that are able to bind to several JWH molecules and metabolites. The skilled person is aware that for these antibodies to recognize JWH molecules they must bind to particular structures or epitopes of the hapten (in this context the hapten being that part of the immunogen that is not the crosslinker or accm); the epitopes are often distinct groups incorporating functional groups.

For example, with reference to the JWH immunogen of structure (a) of Group I, the epitope recognized by the antibody will be all or part of the 3-(1-naphthoyl)-1H-indole moiety.

For an immunogen of structure (b) of Group I the epitope recognized by the antibody will be all or part of the N-pentyl-3-carbonyl-1H-indole moiety.

The accm can be any material that makes all or part of the hapten susceptible to antibody recognition and binding. For example the accm can be a protein, a protein fragment, a synthetic polypeptide or a semi-synthetic polypeptide.

Especially preferred immunogens of the invention correspond to structures (a) and (c) of Group I in which structure (a) has Y=carbonyl, n=0 and D=pentylene and structure (c) has Y=carbonyl, A= O, n=1 and D= methylene.

A further aspect of the invention is an antibody raised against an immunogen of structure (a), (b) or (c) of Group I, that is able to bind to molecules of the JWH family and their metabolites that comprise structure I. The term 'able to bind to', as used herein, does not imply that the antibodies have a choice of whether or not to bind to the JWH molecules, but that under standard immunoassay conditions the antibodies will bind to the JWH molecules. The antibodies are preferably raised against immunogens of structure (a) or (c) of Group I, the antibodies being able to bind to several molecules and metabolites of the JWH family, including JWH-018 and its N-alkyl hydroxylated metabolites. It is especially preferred that the antibodies are raised from structure (a) of Group I in which Y=carbonyl, n=0 and D=pentylene and structure (c) of Group I in which Y=carbonyl, A= O, n=1 and D= methylene, the antibodies able to bind to several molecules and metabolites of the JWH family, including JWH-018 and its dealkylated, hydroxylated and carboxylated metabolites, JWH-073, JWH-081, JWH-200 and JWH-398. Preferably, the antibodies raised against immunogens of structure (a) or (c) of Group I are also able to bind to one or more metabolites of JWH-018 identified as M1 - M5 in Figure 7, namely JWH-018 N-pentanoic acid metabolite (M1), JWH-018 5-hydroxyindole metabolite (M2), JWH-018 4-hydroxyindole metabolite (M3), JWH-018 N-(5-hydroxypentyl) metabolite (M4) and JWH-018 6-hydroxyindole metabolite (M5); and/or the JWH-018 metabolite JWH-018 N-(4-hydroxypentyl) metabolite; and/or the 5-fluoropentyl derivative of JWH-018, 1-(5-fluoropentyl)indol-3-yl (1-naphthyl) methanone; and/or the JWH-018 metabolite identified as JWH-250 in Figure 6; and/or one or more JWH-073 metabolites selected from JWH-073 N-(3-hydroxybutyl) metabolite and JWH-073 N-(4-hydroxybutyl) metabolite. These compounds are available from, for example, Cayman Chemical Company. The antibodies also have the potential to bind to $CB_1$-active derivatives of JWH molecules that could represent future generations of SSCs.

Optionally, the antibodies may have broad cross-reactivity across the JWH family and metabolites. For example, without intending to limit the invention thereto, antibodies raised to Immunogen I (structure (a) of Group I) may be specific to JWH-018 N-(5-hydroxypentyl) metabolite (M4) and cross-reactive to JWH-073, JWH-200, JWH-018, JWH-018 N-pentanoic acid metabolite (M1), JWH-018 5-hydroxyindole metabolite (M2), JWH-018 4-hydroxyindole metabolite (M3), JWH-018 6-hydroxyindole metabolite (M5) and 3-(1-naphthoyl)-1H-Indole. Similarly, antibodies raised to Immunogen II (structure (c) of Group I) may be specific to JWH-018 6-hydroxyindole metabolite (M5) and cross-reactive to JWH-073, JWH-200, JWH-398, JWH-018, JWH-018 N-pentanoic acid metabolite (M1), JWH-018 5-hydroxyindole metabolite (M2), JWH-018 4-hydroxyindole metabolite (M3) and JWH-018 N-(5-hydroxypentyl) metabolite (M4).

Optionally, the antibodies raised to Immunogen I (structure (a) of Group I) will be able to bind to an epitope of JWH-073, JWH-200, JWH-018, JWH-018 N-pentanoic acid metabolite (M1), JWH-018 5-hydroxyindole metabolite (M2), JWH-018 4-hydroxyindole metabolite (M3), JWH-018 N-(5-hydroxypentyl) metabolite (M4) and JWH-018 6-hydroxyindole metabolite (M5). Optionally, the epitope will be all or part of 3-(1-naphthoyl)-1H-Indole.

Further optionally, the antibodies raised to Immunogen II (structure (c) of Group I) will be able to bind to an epitope of JWH-073, JWH-200, JWH-398, JWH-018, JWH-018 N-pentanoic acidmetabolite (M1), JWH-018 5-hydroxyindole metabolite (M2), JWH-018 4-hydroxyindole metabolite (M3), JWH-018 N-(5-hydroxypentyl) metabolite (M4) and JWH-018 6-hydroxyindole metabolite (M5).

The invention also describes an antibody raised against an immunogen of structure (d), (e), (f) or (g) the antibody being able to bind to molecules of the CP family, metabolites of CP molecules and future SSC molecules comprising structure II. When used in reference to an antibody, the word specific in the context of the current invention refers to the analyte that is preferably bound by the antibody, as gauged by a suitable metric such as the $IC_{50}$. Given the $IC_{50}$ of various analytes their cross-reactivities can be calculated. The antibody can either be a polyclonal or monoclonal antibody using well-known methods. If the polyclonal antibody possesses the required specificity and sensitivity, that is, it binds a single analyte within the detection range of the assay, development of a monoclonal antibody is unnecessary. Alternatively, a polyclonal or monoclonal antibody that binds to several analytes might be desirable; in the context of the current invention antibodies that bind several analytes are preferred. One or more antibodies of the invention can be incorporated into a kit for the detection and determination of individual or multiple SSCs. The skilled person in the immunodiagnostic field is aware of several alternative immunoassay formats that could incorporate the antibodies of the invention either in solution or tethered (e.g. covalently bonded or electrostatically 'non-bonded' through van der waal's forces) to a solid substrate such as beads, glass/plastic slides or ceramic chips (a chip defined as a small, planar substrate). A preferred solid substrate onto which the antibodies of the invention are covalently bonded is a chip, preferably a ceramic chip; the word 'biochip' can be used to refer to a chip with antibodies attached. Such a "biochip" is described in EP1273349, incorporated herein by reference its entirety. Thus the invention also provides a solid substrate, preferably a biochip, comprising antibodies raised to an immunogen of one or more of structures (a), (b), (c), (d), (e), (f), (g) or (h), the antibodies being able to bind to an epitope of one or more molecules of the JWH family and/or CP family and/or one or more metabolites thereof. The antibodies of the invention can be used for the detection or determination of a single SSC such as JWH-018, either as the parent molecule or as a metabolite, but a preferred embodiment is the use of one or more antibodies, preferably two or more antibodies, at least one derived from Group I and one derived from Group II, for the detection or determination of several SSCs and/or their metabolites from the JWH and CP families. The detection and determination criteria for a SSC using an immunoassay platform includes, as is well-known in the art, exceeding a pre-defined cut-off/concentration value or measuring the calibrator equivalent value as derived from a calibrator curve (also referred to as a standard curve).

Classification of immunoassays depends on whether one (noncompetitive) or two (competitive) antigens are used:

1. Competitive, homogeneous immunoassay
The antigen in the unknown sample competes with labeled antigen to bind with antibodies. The amount of unbound, labeled antigen is then measured, which is directly proportional to the concentration of sample antigen.
2. Competitive, heterogeneous immunoassay
The antigen in the unknown sample competes with labeled antigen to bind with antibodies. The amount of labeled antigen bound to the antibody site is then measured. In this method, the response will be inversely related to the concentration of antigen in the unknown.
3. One-site, noncompetitive immunoassay
The unknown antigen in the sample binds with labeled antibodies. The unbound, labeled antibodies are washed away, and the bound, labeled is measured, which is directly proportional to the amount of unknown antigen.
4. Two-site, noncompetitive immunoassays
The antigen in the unknown sample is bound to the antibody site, then labeled antibody is bound to the antigen. The amount of labeled antibody on the site is then measured. It will be directly proportional to the concentration of the antigen because labeled antibody will not bind if the antigen is not present in the unknown sample. This type is also known as sandwich assay as the antigen is "sandwiched" between two antibodies.

Another aspect of the invention is a method of detecting or determining synthetic cannabinoids of the JWH family and their metabolites in an *in vitro* sample of an individual or in a solution derived from a substance suspected of containing synthetic cannabinoids comprising: contacting the sample or solution with one or more detecting agents and one or more antibodies of the invention that bind to molecules of the JWH family, measuring the detecting agents, and detecting or determining, by reference to calibrators, the presence or concentration of a molecule or molecules of the JWH family.

Optionally, the method further comprises a step of measuring the detecting agents before detecting or determining, by reference to calibrators, the presence or concentration of a molecule or molecules of the CP family. The detecting agents measuring step can be measuring detecting agent bound to the antibodies, the detecting agent being labeled antigen in a competitive, homogeneous immunoassay in which unbound, labeled antigen is measured. Alternatively, the detecting agents measuring step can be measuring detecting agent bound to the antibodies, the detecting agent being labeled antigen in a competitive, heterogeneous immunoassay in which bound, labeled antigen is measured. Further alternatively, the detecting agents measuring step can be measuring detecting agent bound to the antibodies, the detecting agent being labeled antibodies in a one-site, noncompetitive immunoassay in which bound, labeled antibodies are measured. Still further alternatively, the detecting agents measuring step can be measuring detecting agent bound to the antibodies, the detecting agent being labeled antibody that is bound to the antigen that is, in turn, also bound to the antibody.

With reference to 'detecting or determining', 'detecting' means qualitatively analyzing for the presence or absence of a substance, 'determining' means quantitatively analyzing for the amount of a substance.

Optionally, the detecting agent is a small molecule, generally of similar structure to a molecule to be detected conjugated to a labelling agent, the detecting agent being able to bind to one of the antibodies of the invention. The labelling agent is selected from an enzyme, a luminescent substance, a radioactive substance, or a mixture thereof. Preferably, the labelling agent is an enzyme, preferably a peroxidase, more preferably horseradish peroxidase (HRP). Alternatively, or additionally, the luminescent substance may be a bioluminescent, chemiluminescent or fluorescent material. For the purposes of the invention, the patient sample to be used for *in vitro* analysis can be hair or a peripheral biological fluid but is preferably whole blood, serum, plasma, or urine.

Preferably the synthetic cannabinoids to be detected or determined are one or more of JWH-018, JWH-073, JWH-200 and JWH-398 and the one or more antibodies are derived from immunogens of structures (a) and (c) of Group I. When referring to the detection or determination of a JWH molecule, with or without a suffixed number attached to JWH and CP, the metabolite or metabolites are also inferred unless otherwise stated.

The immunogen of structure (a) preferably has a crosslinker -X-Y- in which Y is carbonyl and is attached to the accm, and X is pentylene, and the immunogen of structure (c) has a crosslinker -O-CH2-C(O)- in which the carbonyl is attached to the accm.

The invention also describes kits for detecting or determining a molecule or molecules of the JWH family comprising one or more antibodies of the invention. Preferably, the kit comprises one or more antibodies raised to an immunogen of either structure (a), (b), (c) or (d) of Group I. More preferably the antibodies of the kit are derived from an immunogen of structure (a) and/or (c). A kit comprising antibodies derived from Immunogens I or II (Figure 5) for detecting or determining one or more of JWH-018, JWH-073, JWH-200 and JWH-398 is particularly preferred. Optionally, the kit may comprise antibodies derived from Immunogens I or II for additionally or alternatively detecting or determining one of more of JWH-081, JWH-018 N-pentanoic acid metabolite (M1), JWH-018 5-hydroxyindole metabolite (M2), JWH-018 4-hydroxyindole metabolite (M3), JWH-018 N-(5-hydroxypentyl) metabolite (M4), JWH-018 6-hydroxyindole metabolite (M5), JWH-018 N-(4-hydroxypentyl) metabolite, 1-(5-fluoropentyl)indol-3-yl (1-naphthyl) methanone, JWH-250, JWH-073 N-(3-hydroxybutyl) metabolite and JWH-073 N-(4-hydroxybutyl) metabolite.

The antibodies of the kit are preferably tethered to any suitable solid support such as a chip. Although the solid support can be of any suitable shape such as a bead or a slide and of any suitable material such as silicon, glass or plastic, the solid support is preferably a ceramic chip. The kit may further include calibrators and one or more detecting agents and optionally includes instructions for the use of the antibodies of the kit and if incorporated, the calibrators and detecting agents, for detecting and determining molecules from the JWH CP family. The invention also embodies solid supports comprising the novel antibodies.

The antibodies of the invention are used for the detection or determination of JWH molecules either in herbal mixtures, an *in vitro* sample taken from an individual or any other substance suspected of their incorporation. A preferred use of the antibodies of the invention is their use in the detection and/or quantification of JWH-018, JWH-073, JWH-200 and JWH-398 in herbal mixtures and/or JWH-073, JWH-200, JWH-398 and JWH-018 and its metabolites in *in vitro* samples taken from individuals.

## General Methods, Examples and Results

*<u>Preparation of Haptens, Immunogens and Detecting Agents</u>*

Although haptens provide defined structural epitopes, they are not in themselves immunogenic and therefore need to be conjugated to carrier materials, which will elicit an immunogenic response when administered to a host animal. Appropriate carrier materials commonly contain poly(amino acid) segments and include polypeptides, proteins and protein fragments. Illustrative examples of useful carrier materials are bovine serum albumin (BSA), egg ovalbumin, bovine gamma globulin, bovine thyroglobulin (BTG), keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. Also, carbohydrates, yeasts or polysaccharides may be conjugated to the hapten to produce an immunogen.

The haptens can also be coupled to a detectable labelling agent such as an enzyme (for example, horseradish

peroxidase), a substance having fluorescent properties or a radioactive label for the preparation of detecting agents for use in the immunoassays. The fluorescent substance may be, for example, a monovalent residue of fluorescein or a derivative thereof.

Immunogen formation for the invention described herein involves conventional conjugation chemistry. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS).

General Procedure for MALDI-TOF Analysis of Immunogens.

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1μl) were analysed using a matrix of sinapinic acid and bovine serum albumin (Fluka) was used as an external calibrant.

Preparation of Antisera

In order to generate polyclonal antisera, an immunogen of the present invention is mixed with Freund's adjuvant and the mixture is injected into a host animal, such as rabbit, sheep, mouse, guinea pig or horse. Sheep are the preferred host animal. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding.

Immunoassay Development

The process of developing an immunoassay is well known to the person skilled in the art. Briefly, for a competitive immunoassay in which the target analyte is a non-immunogenic molecule such as a hapten, the following process is conducted: antibodies are produced by immunising an animal, preferably a mammalian animal, by repeated administration of an immunogen. The serum from the immunised animal is collected when the antibody titre is sufficiently high. A detecting agent is added to a sample containing the target analyte and the raised antibodies, and the detecting agent and analyte compete for binding to the antibodies. The process may comprise fixing said serum antibodies to a backing substrate such as a polystyrene solid support or a ceramic chip. The antibodies can be polyclonal or monoclonal using standard techniques. The signal emitted in the immunoassay is proportionate to the amount of detecting agent bound to the antibodies which in turn is inversely proportionate to the analyte concentration. The signal can be detected or quantified by comparison with a calibrator.

**Examples**

Preparation of Hapten A (see Figure 1)

Example - 1: Preparation of 3-(1-naphthoyl)-1H-indole **1**

To a cooled solution of indole (5.85g, 50mmol) in ether (50ml) under nitrogen was added slowly a solution of methylmagnesium bromide (3M) in ether (17.5ml). After addition, the reaction mixture was warmed up to room temperature and stirred for 2h at room temperature. Then the mixture was cooled down again to 0°C, and to it was added slowly with stirring a solution of 1-naphthoylchloride (9.5g, 50mmol) in ether (50ml). The resulting mixture was warmed up to room temperature and stirred for 2h at room temperature followed by slow addition of saturated ammonium chloride solution (375ml). The mixture was then stirred overnight at room temperature. A white solid was formed, filtered, washed by ether and dried under high vacuum to give 3-(1-naphthoyl)-1H-Indole **1** (12.3g, 91%).

**Example 2**: Preparation of N-(5-Ethoxycarbonylpentyl)-3-(1-naphthoyl)-1H-indo le **2**

To a suspension of sodium hydride (1.1g, 30mmol, 60% in mineral oil) in DMF (100ml) under nitrogen was added solid 3-(1-naphthoyl)-1H-indole **1** (5.43g, 20mmol). After stirring at room temperature for 1h, a solution of ethyl 6-bromohexanoate (6.6g, 30mmol) in DMF (10ml) was added slowly with stirring over a period of 15 min and the mixture was then heated at 60°C for 3h. The solvent was removed under high vacuum and the crude product was suspended

in water (150ml) and extracted by ethyl acetate (2x150ml). The combined ethyl acetate phases were washed by water (1x100ml), brine (1x100ml), dried over sodium sulphate filtered and concentrated to dryness. The crude product obtained was purified by chromatography on silica gel using hexane/ethyl acetate (8/2) to give the title compound **2** as an oil which became solid in the cold (7.1g, 86%).

**Example 3**: Preparation of N-(5-carboxypentyl)-3-(1-naphthoyl)-1H-indole (Hapten-A)

To a solution of 2 (5.0g, 12mmol) in a mixture of THF/H2O (1:1) was added potassium hydroxide (1.7g) and the mixture was stirred at 60 4°C for 1h. The THF was removed under vacuum, the aqueous solution acidified to pH 1 by the addition of hydrochloric acid solution (1N) and extracted by ethyl acetate (3x100ml). The combined organic phases were washed by water (100ml), brine solution (100ml), dried over sodium sulphate, filtered and concentrated to dryness. The crude product obtained was dissolved in ethyl acetate (10ml) and the Hapten-A precipitated by the addition of a mixture of ether/hexane as a white solid, filtered and dried under high vacuum to give Hapten-A (3.6g, 78%).

**Example 4**: Conjugation of Hapten-A to BSA

To a solution of Hapten-A (52.2mg, 0.13mmol) in DMF (1.0ml) was added N,N-dicyclohexylcarbodiimide (DCC) (34.0mg, 0.16mmol) and N-hydroxysuccinimide (19.0mg, 0.16mmol) and the mixture was stirred at room temperature overnight. The dicyclohexylurea formed was removed by filtration and the solution added dropwise to a solution of BSA (200mg, 3.0□mol) in 50mM sodium bicarbonate solution (pH 8.5) (10ml). The mixture was then stirred overnight at 4°C. The solution was then dialysed against 50mM phosphate buffer pH 7.2 (3 changes) for 24h at 4°C, and freeze-dried. MALDI results showed 9.83 molecule of Hapten-A had been conjugated to one molecule of BSA.

**Example 5**: Conjugation of Hapten-A to BTG (Immunogen I)

To a solution of Hapten-A (58.0mg, 0.15mmol) in DMF (1.0ml) was added N,N-dicyclohexylcarbodiimide (DCC) (34.0mg, 0.165mmol) and N-hydroxysuccinimide (19.0mg, 0.16mmol) and the mixture was stirred at room temperature overnight. The dicyclohexylurea formed was removed by filtration and the solution was added dropwise to a solution of BTG (150mg) in 50mM sodium bicarbonate solution (pH 8.5) (10ml). The mixture was then stirred overnight at 4°C. The solution was then dialysed against 50mM phosphate buffer pH 7.2 (3 changes) for 24h at 4°C, and freeze-dried to give Immunogen I.

**Example 6**: Conjugation of Hapten-A to HRP

EDC hydrochloride (10mg) was dissolved in water (0.5ml) and immediately added to a solution of Hapten-A (2mg) in DMF (0.2ml). After mixing, this solution was added dropwise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP detecting agent was then dialysed overnight against 10L of PBS at pH 7.2 at 4°C.

Preparation of Hapten B (see Figure 2)

**Example 7**: Preparation of 5-methoxy-3-(1-naphthoyl)-1H-indole **4**

To a cooled solution at 0°C of 5-methoxy-1H-indole **3** (7.4g, 50mmol) in diethyl ether (150ml) under nitrogen was added dropwise a solution of methylmagnesium bromide (3M) in diethyl ether (17.5ml) and the mixture was stirred for 2h at room temperature. The solution was then cooled at 0°C and to this solution was added a solution of 1-naphthoylchloride (9.5g, 50mmol) in diethyl ether (100ml) dropwise over a period of 15 min. After the addition was completed the solution was warmed up at room temperature and stirred for 2h followed by slow addition of saturated ammonium chloride solution (375ml) and stirred overnight. The white solid formed was filtered, washed by ether and dried under high vacuum to give 5-methoxy-3-(1-naphthoyl)-1H-indole **4** (11.3g, 75%).

**Example 8**: Preparation of 5-methoxy-3-(1-naphthoyl)-N-pentyl-1H-indole **5**

To a suspension of sodium hydride (1.54g, 45.7mmol, 60% in mineral oil) in DMF (100ml) under nitrogen was added dropwise a solution of 5-methoxy-3-(1-naphthoyl)-1H-indole **4** (9.83g, 32.6mmol) in DMF (50ml) and the mixture was stirred at 40°C for 1h. The solution was then cooled to room temperature and to this mixture was added a solution of 1-bromopentane (8.2g, 54.3mmol) in DMF (25ml). The mixture was stirred at 60°C for 1h. The solvent was removed under high vacuum and the crude product was suspended in water (200ml) and extracted by ethyl acetate (2x200ml).

The combined ethyl acetate phases were washed by water (1x100ml), brine (1x100ml), dried over sodium sulphate, filtered and concentrated to dryness. The crude product obtained was purified by chromatography on silica gel using hexane/ethyl acetate (7/3) to give the title compound **5** as an oil which solidified upon cooling (7.1g, 59%).

**Example 9**: Preparation of 5-hydroxy-3-(1-naphthoyl)-N-pentyl-1H-indole **6**

To a solution of hydrobromic acid (48 w/w/%) in water (150ml) was added **5** (6.5g, 17.5mmol) and the mixture was heated at reflux for 3h. The solution was cooled to room temperature and concentrated to dryness. Water was then added (200ml), the solution neutralized to pH 7-8 and then extracted with ethyl acetate (3x150ml). The combined organic layers were washed by water (150ml), brine (150ml), dried over $Na_2SO_4$, filtered and concentrated to dryness. The crude product was then recrystallized from ethyl acetate / hexane to give a white solid of 5-hydroxy-3-(1-naphthoyl)-N-pentyl-1H-indole **6** (4.5g, 72%).

**Example 10**: Preparation of 5-(tert-butoxycarbonylmethoxy)-3-(1-naphthoyl)-N-pentyl-1H-indole **7**

To a suspension of sodium hydride (452mg, 13.4mmol, 60% in mineral oil) in DMF (25ml) under nitrogen was added dropwise a solution of **6** (3.7g, 10.35mmol) in DMF (50ml) and the mixture was stirred at 60°C for 1h. The solution was then cooled to room temperature and to this mixture was added a solution of tert-butyl bromoacetate (2.62g, 13.4mmol) in DMF (25ml). The mixture was stirred at 60°C for 3h. The DMF was removed under high vacuum and the crude product was suspended in water (100ml) and the mixture was extracted by ethyl acetate (2x100ml). The combined ethyl acetate phases were washed by water (1x50ml), brine (1x50ml), dried over sodium sulphate filtered and concentrated to dryness. The crude product obtained was purified by chromatography on silica gel using hexane/ethyl acetate (9/1) to give the title compound 7 (3.5g, 72%).

**Example 11**: Preparation of 5-carboxymethoxy-3-(1-naphthoyl)-N-pentyl-1H-indole (Hapten-B)

To a solution of 7 (3.0g, 6.4mmol) in dichloromethane (50ml) was added TFA (25ml) and the mixture was stirred at room temperature for 3h. The mixture was evaporated to dryness and the crude obtained was purified by chromatography on silica-gel using 5% methanol in chloroform to give Hapten-B (2.1g, 79%). NMR $^{13}C$ ($CD_3OD$, $\delta$ ppm): 194.55, 173.21, 156.53, 141.14, 140.15, 135.25, 134.10, 131.98, 131.17, 129.46, 128.9, 1127.85, 127.44, 126.87, 125.55, 125.85, 117.92, 115.14, 112.72, 106.33, 65.54, 30.59, 29.87, 23.2, 14.25

**Example 12**: Conjugation of Hapten-B to BSA

To a solution of Hapten-B (62.4mg, 0.15mmol) DMF (1.0ml) was added N,N-dicyclohexylcarbodiimide (DCC) (34.1mg, 0.16mmol) and N-hydroxysuccinimide (19.02mg, 0.16mmol) and the mixture was stirred at room temperature overnight. The dicyclohexylurea formed was removed by filtration and the solution was added dropwise to a solution of BSA (200mg, 3□mol) in 50mM sodium bicarbonate solution (pH 8.5) (10ml). The mixture was then stirred overnight at 4°C. The solution was then dialysed against 50mM phosphate buffer pH 7.2 (3 changes) for 24h at 4°C, and freeze-dried. MALDI results showed 37.2 molecules of Hapten-B had been conjugated to one molecule of BSA.

<u>Preparation of Hapten C (see Figure 3)</u>

**Example 13**: Conjugation of Hapten-B to BTG (Immunogen II)

To a solution of Hapten-B (56.0mg, 0.13mmol) in DMF (1.0ml) was added N,N-dicyclohexylcarbodiimide (DCC) (30.6mg, 0.14mmol) and N-hydroxysuccinimide (17.1mg, 0.14mmol) and the mixture was stirred at room temperature overnight. The dicyclohexylurea formed was removed by filtration and the solution was added dropwise to a solution of BTG (150mg) in 50mM sodium bicarbonate solution (pH 8.5) (15ml). The mixture was then stirred overnight at 4°C. The solution was then dialysed against 50mM phosphate buffer pH 7.2 (3 changes) for 24h at 4°C, and freeze-dried to give Immunogen II.

**Example 14**: Preparation of 3-carboxy-1-pentyl-1H-indole (Hapten-C)

Indole 3-carboxylic acid (3g, 18.62mmol) was added to a suspension of sodium hydride (60% in oil) (1.11g, 1.5eq) in dry DMF (30ml) under a nitrogen atmosphere. The mixture was stirred at room temperature for 45 min ($H_2$ evolving has ceased) and to this was added 1-bromopentane (4.62ml, 2eq) in dry DMF (10ml) dropwise. The mixture was stirred at room temperature overnight. The solvents were removed *in vacuo* and to the residue was added water (30ml) and ethyl acetate (30ml). The ethyl acetate portion was separated, dried over sodium sulphate, filtered and

evaporated to dryness. The crude residue was purified by column chromatography (silica gel: 20% ethyl acetate in hexane) to give the title compound (2.12g, 49%) as a cream solid.

**Example 15**: Conjugation of 3-carboxy-1-pentyl-1H-indole (Hapten C) to BSA

To a solution of Hapten-C (26.13mg , 0.113mmol) in DMF (1.0ml) was added N,N-dicyclohexylcarbodiimide (DCC) (25.64, 0.1243 mmol) and N-hydroxysuccinimide (14.30mg, 0.1243 mmol) and the mixture was stirred at room temperature overnight. The dicyclohexylurea formed was removed by filtration and the solution was added dropwise to a solution of BSA (150mg) in 100mM sodium bicarbonate solution (pH 8.5) (9ml). The mixture was then stirred overnight at 4°C. The solution was then dialysed against 50mM phosphate buffer pH 7.2 (3 changes) for 24 hours at 4°C, and freeze-dried.

**Example 16**: Conjugation of 1-pentyl-1-H-indole-3-carboxylic acid (3-carboxy-1-pentyl-1H-indole, Hapten C) to BTG (now renamed as Immunogen VIII)

To a solution of Hapten-C (31.22mg, 0.135mmol) in DMF (1.0ml) was added N,N-dicyclohexylcarbodiimide (DCC) (30.74mg, 0.149mmol) and N-hydroxysuccinimide (17.1mg, 0.149mmol) and the mixture was stirred at room temperature overnight. The dicyclohexylurea formed was removed by filtration and the solution was added dropwise to a solution of BTG (150mg) in 100mM sodium bicarbonate solution (pH 8.5) (15ml). The mixture was then stirred overnight at 4°C. The solution was then dialysed against 50mM phosphate buffer pH 7.2 (3 changes) for 24 hours at 4°C, and freeze-dried to give Immunogen VIII.

**Example 17**: Conjugation of Hapten-B to HRP

EDC hydrochloride (10mg) was dissolved in water (0.5ml) and immediately added to a solution of Hapten-B (2mg) in DMF (0.2ml). After mixing, this solution was added dropwise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP detecting agent was then dialysed overnight against 10L of PBS at pH 7.2 at 4°C.

**Example 18**: Preparation of antibodies to Immunogens I and II.

Aqueous solutions of the immunogens prepared in examples 5 and 13 were formulated with Freund's Complete Adjuvant (FCA) to form emulsions consisting of 2mg/ml Immunogen I and 2mg/ml of Immunogen II in 50%(v/v) FCA. Three sheep were immunised with each emulsion (1° immunisations), 0.25ml being intramuscularly injected at four sites in the rump of each animal. Subsequent immunisations (boosts 2-8) contained 1mg/ml Immunogen I and 1mg/ml Immunogen II. All boosts were emulsified in 50%(v/v) Freund's Incomplete Adjuvant (FIA) and administered to the appropriate sheep in the same manner as the 1° immunisations, at monthly intervals. Blood sampling took place 7-14 days after each boost. Each sample was processed to produce antiserum, which was further purified by caprylic acid and ammonium sulphate precipitation to yield an immunoglobulin (Ig) fraction. The Ig fraction was evaluated by competitive ELISA microtiter plate assay, as described in example 19 below.

**Example 19**: Development of competitive ELISA for JWH synthetic cannabinoids and metabolites.

(a) The wells of an enhanced binding 96 well polystyrene microtiter plate were coated with the Ig fraction of the antiserum raised to Immunogen I (Hapten A-BTG - example 5) and diluted in 10mM Tris, pH8.5 (125μl/well). The appropriate antibody coating concentration was determined using standard ELISA checkerboard techniques. The plate was incubated for 2 hours at 37°C, washed 4 times over a 10-minute period with Tris buffered saline containing Tween 20 (TBST) and tapped dry. Standard solutions of JWH synthetic cannabinoids, metabolites and selected molecules were prepared in TBST and 50μl of each was added to the appropriate wells. 75μl of conjugate (Hapten A-HRP) diluted in Tris buffer containing EDTA, D-mannitol, sucrose, thimerosal and BSA, was added to each of the wells. The appropriate dilution of conjugate was also determined using standard ELISA checkerboard techniques. The plate was incubated at 25°C for 1 hour. The excess unbound conjugate was removed by washing 6 times over a 10-minute period with TBST and tapped dry. 125μl of tetramethylbenzedine (TMB) substrate solution was added to each well of the plate that was then incubated for 15-20 minutes in the dark at room temperature. The reaction was terminated by addition of 125μl of 0.2M sulphuric acid to each well. The absorbance was then measured at 450nm using a microtiter plate reader. Employing each series of standards, calibration curves were generated and these were used to determine the specificity of the immunoassay for the JWH synthetic cannabinoids, metabolites and selected molecules. The results of this study are presented in Tables 1 to 3, cross-reactivity being calculated

according to the following formula:

$$\%CR = IC_{50,\ JWH\text{-}018} / IC_{50,\ CR}\ X\ 100$$

Where %CR is the percentage cross-reactivity, $IC_{50,\ YWH\text{-}018}$ is the concentration of JWH-018 that causes 50% displacement of signal and $IC_{50,\ CR}$ is the concentration of JWH synthetic cannabinoid/metabolite/selected molecule that causes 50% displacement of signal.

**Table 1:** Data generated from a competitive microtiter plate assay for JWH synthetic cannabinoids and metabolites, employing antiserum raised to Immunogen II (Hapten B-BTG) and conjugate (Hapten A-HRP) as detection reagent. (b) In a similar manner to that described in Example 19(a), the wells of a 96 well microtiter plate were coated with the Ig fraction of the antiserum raised to Immunogen II (Hapten A-BTG). Conjugate (Hapten A-HRP) was employed as a detection reagent. The data generated is presented in Table 2.

**Table 2**: Data generated from a competitive microtiter plate assay for JWH synthetic cannabinoids and metabolites, employing antiserum raised to Immunogen I (Hapten A-BTG) and conjugate (Hapten A-HRP) as detection reagent.

(c) The resulting competitive ELISA for synthetic cannabinoids and metabolites was further employed to analyse urine and serum samples from 20 patients (**Table 3**).

(d) The resulting competitive ELISA for synthetic cannabinoids and metabolites was also employed to test for the 5-fluoropentyl derivative of JWH-018, (1-(5-fluoropentyl)indol-3yl (1-naphthyl) methanone (**Table 4**).

**Results**

**Table 1** Antibody characterisation using antiserum raised to Immunogen II and detecting agent derived from Hapten-A in a competitive assay format (CR based on 100% for JWH-018)

| Analyte | $IC_{50}$ ng/ml | % Cross-reactivity |
|---|---|---|
| JWH-018 | 2.11 | 100.00 |
| JWH-073 | 1.56 | 135.26 |
| JWH-398 | 17.55 | 12.02 |
| JWH-200 | 1.66 | 127.11 |
| 3-(1-naphthoyl)-1H-Indole | >> 40 | << 5.28 |
| M1 | 5.48 | 38.50 |
| M2 | 1.62 | 130.25 |
| M3 | 9.16 | 23.03 |
| M4 | 1.15 | 183.48 |
| M5 | 0.98 | 215.31 |

» implies a value greatly exceeding the value given (40 ng/ml was the highest concentration tested)
« implies a value greatly below the value given

**Table 2** Antibody characterisation using antiserum raised to Immunogen I and detecting agent derived from Hapten-A in a competitive assay format (CR based on 100% for JWH-018)

| Analyte | $IC_{50}$ ng/ml | % Cross-reactivity |
|---|---|---|
| JWH-018 | 2.71 | 100.00 |
| JWH-073 | 0.93 | 291.40 |
| JWH-398 | >> 40 | << 6.78 |
| JWH-200 | 0.31 | 874.19 |
| 3-(1-naphthoyl)-1H-Indole | 3.84 | 70.57 |
| M1 | 0.42 | 645.24 |
| M2 | 0.39 | 694.87 |

(continued)

| Analyte | $IC_{50}$ ng/ml | % Cross-reactivity |
|---|---|---|
| M3 | 25.51 | 10.62 |
| M4 | 0.18 | 1505.56 |
| M5 | 2.04 | 132.84 |

>> implies a value greatly exceeding the value given (40 ng/ml was the highest concentration tested)
<< implies a value greatly below the value given

**Table 3** Sensitivity and cross-reactivity (CR) of antibodies raised to Immunogens I & II of selected molecules

| Analyte | Standard Conc$^n$ ng/ml | $IC_{50}$ ng/ml | % CR |
|---|---|---|---|
| Serotonin | 750.00 | > 750.00 | < 0.28 |
| 4-Methoxypsilocin | 750.00 | > 750.00 | < 0.28 |
| Delta-9-THC | 750.00 | > 750.00 | < 0.28 |
| Cannabinol | 750.00 | > 750.00 | < 0.28 |
| 11-Hydroxy-δ-9-THC | 750.00 | > 750.00 | < 0.28 |
| CP 47,497 | 750.00 | > 750.00 | < 0.28 |
| 3-Carboxy-N-pentyl-1H -indole | 750.00 | > 750.00 | < 0.28 |
| 3-Carboxy-1H-indole | 750.00 | > 750.00 | < 0.28 |
| 3-Carboxymethyl-5-hydroxy-1H-indole | 750.00 | > 750.00 | < 0.28 |
| 5-Hydroxytryptophol | 750.00 | > 750.00 | < 0.28 |

**Table 4** Sensitivity and cross-reactivity (CR) of antibodies raised to Immunogens I & II of JWH-018 and 1-(5-Fluoropentyl)indol-3-yl (1-naphthyl) methanone* (5-Fluoropentyl derivative)

| Immunogen | | $IC_{50}$ ng/ml | % Cross-reactivity |
|---|---|---|---|
| I | JWH-018 | 2.30 | 100.00 |
| I | 5-Fluoropentyl derivative | 0.02 | 11500.00 |
| II | JWH-018 | 1.97 | 100.00 |
| II | 5-Fluoropentyl derivative | 0.22 | 908.00 |

* Binding of this molecule to the $CB_1$ receptor is detailed in US6900236

Immunoassays using antibodies of the invention to test for potential cross-reactants (Table 3) and to screen the urine and serum of twenty patients for cross-reactive molecules, did not reveal any cross-reactants which could invalidate the measurements taken using the antibodies, methods, kits and products of the invention.

As can be seen from Tables 1 to 4, for the first time antibodies have been provided that bind to various JWH molecules, metabolites and potential metabolites, whereas other common indole-containing molecules and non-JWH $CB_1$-active molecules do not bind. The antibody produced from Immunogen I is able to bind to JWH molecules known to be incorporated in herbal therapeutics such as JWH-018, proposed metabolites such as M2 (Wintermeyer et al 2010), and potential metabolites such as M5. The detection and quantification of other JWH SSCs is also provided for, the antibodies of the invention bind a range of molecules comprising the 3-(1-naphthoyl)-1H-indole structure (e.g. JWH-071, JWH-398, M1 etc.). Table 3 confirms that the same antibodies do not cross-react with other psychoactive drugs, with molecules present in biological samples of patients who have not taken JWH containing substances or with molecules without the 3-(1-naphthoyl)-1H-indole such as 3-carboxy-1H-indole. The concept of using the antibodies of the invention to detect and determine future stealth synthetic cannabinoids is highlighted in Table 4; the 5-fluoropentyl derivative known to bind the CB1 receptor, but which as of yet has not been detected in herbal therapeutics or similar substances, binds to antibodies raised from Immunogens I and II.

Preparation of Hapten D / Immunogen IV (see Figure 4)

**Example 20**: Preparation of 4-methoxy-1-naphthoic acid **2**.

4-Methoxy-1-naphthaldehyde (10g, 53.72mmol) was dissolved in *t*-butanol (75ml) and 2-methyl-2-butene (35ml) was added at room temperature. A solution of sodium chlorite (7.99g, 70.63mmol) and sodium dihydrogen orthophosphate (9.15g, 76,27mmol) in water (50ml) was added dropwise. The reaction mixture was stirred at room temperature overnight. The reaction mixture was concentrated in vacuo and the residue was washed with a solution of 2MHCl (100ml) and the solid was filtered and washed with dichloromethane (100ml) and water (100ml) and dried in vacuo. The solid was recrystallised from methanol to give 5.95g (55%) of 4-methoxy-1-naphthoic acid **2** as cream crystals.

**Example 21**: Preparation of 3-(4-methoxy-1-naphthyoyl)indole **3**

Indole (2.30g, 19.65mmol) was dissolved in diethylether (50ml) under nitrogen atmosphere and cooled to 0°C. 3M MeMgBr solution (6.88ml, 20.63mmol) was added dropwise and allowed to stir at room temperature for 3 hours. In the meantime, 4-methoxy-1-naphthoic acid **2** (3.97g, 19.65mmol) was dissolved in thionyl chloride (15ml) and refluxed for 90 min, then the solvent was removed under vacuo and the residue was dissolved in diethylether (25ml). The resulting solution was added dropwise to the indole reaction mixture at 0°C and then allowed to stir at room temperature overnight. A solution of ammonium chloride (200ml) was added to the reaction mixture and the resulting mixture was stirred at room temperature for 3 hours. The solid that was formed was collected by filtration, washed with water and hexane then air dried. The resulting solid was triturated with ether to give 4.78g (81%) of 3-(4-methoxy-1-naphthyoyl)indole **3** as a cream solid.

**Example 22**: Preparation of 1-N-pentyl-3-(-4-methoxy-1-naphthyloyl)indole **4**

3-(4-Methoxy-1-naphthyoyl)indole **3** (4.78g, 15.86mmol) was suspended in dimethylformamide (35ml) and 60% sodium hydride in mineral oil (0.95g, 23.82mmol) was added portionwise at room temperature, allowing to stir further 40 min at room temperature after the addition was finished. 1-Bromopentane (3.59g, 23.82mmol) was added dropwise at room temperature and the reaction mixture was stirred at room temperature for 3 hours. Reaction was stopped by the addition of water (20ml) and then diluted with ethyl acetate (200ml) and water (150ml). The layers were separated and the organic layer was washed 2 more times with water (2x150ml). The aqueous fractions were combined and extracted 3 times with ethyl acetate (3x100ml). All the organic fractions were combined, dried over sodium sulphate and concentrated in vacuo. The residue was purified by column chromatography (silica gel, 25%-30% ethyl acetate in hexane) to give 3.77g (64%) of 1-N-pentyl-3-(-4-methoxy-1-naphthyloyl)indole **4** as an off yellow brittle solid.

**Example 23**: Preparation of 4-hydroxynaphthalen-1-yl-(1-pentylindol-3-yl) methanone **5**

1-N-pentyl-3-(-4-methoxy-1-naphthyloyl)indole **4** (5.55g, 14.82mmol) was dissolved in anhydrous dichloromethane (40ml) and cooled at -78°C. A solution of 1N$BBr_3$ (16.3ml, 16.3mmol) was added dropwise and once the addition was complete the reaction mixture was allowed to warm up to room temperature and was stirred at room temperature overnight. The reaction mixture was poured into a solution of 2NHCl (100ml) and stirred for 30 min, then extracted three times with dichloromethane (3x150ml) and the combined organic fractions were filtered through celite and washed with dichloromethane. The filtrate was dried over sodium sulphate and concentrated in vacuo. The residue was purified by column chromatography (Biotage Isolera 4, SNAP-100g, 30%-35% ethyl acetate in heptane) to give 840mg (16%) of 4-hydroxynaphthalen-1-yl-(1-pentylindol-3-yl) methanone **5** as a cream solid.

**Example 24**: Preparation of 4-(t-butyloxyacetate)naphthalen-1-yl-(1-pentylindol-3-yl) methanone 6

4-Hydroxynaphthalen-1-yl-(1-pentylindol-3-yl) methanone **5** (840mg, 2.33mmol) was dissolved in acetonitrile (50ml) followed by the addition of potassium carbonate (0.98g, 7.00mmol) and t-butyl bromoacetate (0.68g, 3.50mmol). The reaction mixture was stirred at room temperature overnight. The reaction mixture was filtered and the filtrate was concentrated in vacuo. The residue was purified by column chromatography (Biotage Isolera 4, SNAP-50g, 15% ethyl acetate in hexane) to give 980mg (92%) of 4-(t-butyloxyacetate)naphthalen-1-yl-(1-pentylindol-3-yl) methanone **6** as an orange oil.

**Example 25**: Preparation of 4-carboxymethylether naphthalene-1-yl-(1-pentylindol-3-yl)methanone (Hapten-1)

4-(t-butyloxyacetate)naphthalen-1-yl-(1-pentylindol-3-yl) methanone **6** (980mg, 2.07mmol ) was dissolved in dichloromethane (30ml) and trifluoroacetic acid (30ml) was added and the reaction mixture was stirred for 3 hours at room temperature. The solvent was removed in vacuo and the residue was purified by column chromatography (Biotage

Isolera 4, SNAP-50g, 50% ethyl acetate in hexane) to give 480mg (44%) of 4-carboxymethylether naphthalene-1-yl-(1-pentylindol-3-yl)methanone **(Hapten-1)** as a white solid.

NMR $^{13}C$ (DMSO, $d_7$) (δ: ppm): 189.83, 169.26, 153.99, 138.24, 136.21, 130.99, 130.88, 126.83, 126.71, 126.05, 125.20, 124.79, 124.49, 122.61, 121.69, 121.36, 121.21, 115.63, 110.35, 103.52, 63.12, 45.54, 28.56, 28.41, 21.05, 13.36. (See **figure-5**)

**Example 26**: Conjugation of 4-carboxymethylether naphthalene-1-yl- (1-pentylindol-3-yl)methanone (Hapten - 1) to BSA (Immunogen)

To a solution of Conjugation of 4-carboxymethylether naphthalene-1-yl- (1-pentylindol-3-yl) methanone (Hapten-1) (47.72mg, 0.09mM) in DMF (1.0ml) was added N, N-dicyclohexylcarbodiimide (DCC) (20.42mg, 0.099mM) and N-hydroxysuccinimide (11.39mg, 0.099mM) and the mixture was stirred at room temperature overnight. The dicyclohexylurea formed was removed by filtration and the solution was added drop-wise to a solution of BSA (150mg, 2.3□M) in 50mM sodium bicarbonate solution (pH 8.5) (10ml). The mixture was then stirred overnight at 4° C. The solution was then dialysed against 50mM phosphate buffer pH 7.2 (3 changes) for 24 hours at 4° C, and freeze-dried to give an **Immunogen-IV**.

MALDI results showed 18.16 molecules of **hapten-1** had been conjugated to one molecule of BSA.

**Example 27**: Conjugation of 4-carboxymethylether naphthalene-1-yl- (1-pentylindol-3-yl)methanone (Hapten-D) to BTG (Immunogen-IV)

To a solution of 4-carboxymethylether naphthalene-1-yl- (1-pentylindol-3-yl)methanone (Hapten - D) (53.47mg, 0.101mM) in DMF (1.0ml) was added N, N-dicyclohexylcarbodiimide (DCC) (22.9mg, 0.111mM) and N-hydroxysuccinimide (12.77mg, 0.111mM) and the mixture was stirred at room temperature overnight. The dicyclohexylurea formed was removed by filtration and the solution was added drop wise to a solution of BTG (150mg) in 50mM sodium bicarbonate solution (pH 8.5) (10ml). The mixture was then stirred overnight at 4° C. The solution was then dialysed against 50mM phosphate buffer pH 7.2 (3 changes) for 24 hours at 4° C, and freeze-dried to give **immunogen-IV**

**Example 28**: Conjugation of 4-carboxymethylether naphthalene-1-yl- (1-pentylindol-3-yl)methanone (Hapten - D) to HRP

EDC hydrochloride (10mg) was dissolved in water (0.5ml) and immediately added to a solution of 4-carboxymethylether naphthalene-1-yl- (1-pentylindol-3-yl)methanone (**Hapten-D**) (2mg) in DMF (0.2ml). After mixing, this solution was added drop wise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4° C.

Preparation of Hapten E / Immunogen VII (see Figure 10)

**Example 29**: Preparation of (2-methyl-1H-indol-3-yl)(naphthalen-1-yl) methanone **2**.

2-Methylindole **1** (10g, 76.3mmol) was dissolved in diethylether (100ml) under nitrogen atmosphere and cooled to 0°C. MeMgBr (3M) solution (26.7ml, 80.15mmol) was added dropwise and allowed to stir at room temperature for 3 hours. In the meantime, 1-naphthoic acid (13.13g, 76.3mmol) was dissolved in thionyl chloride (50ml) and refluxed for 90 min, then the solvent was removed under vacuo and the residue was dissolved in diethylether (50ml). The resulting solution was added dropwise to the indole reaction mixture at 0°C and then allowed to stir at room temperature for 2 hours. A solution of ammonium chloride (200ml) was added to the reaction mixture and the resulting mixture was stirred at room temperature overnight. The solid that was formed was collected by filtration, washed with water and hexane then dried in a dessicator over phosphorous pentoxide to give 9.7g (45%) (2-methyl-1H-indol-3-yl)(naphthalen-1-yl) methanone **2** as a cream solid.

**Example 30:** Preparation of ethyl 4-(3-(1-naphthoyl)-2-methyl-1H-indol-1-yl) butanoate **3**

60% Sodium hydride in mineral oil (266mg, 6.65mmol) was suspended in dimethylformamide (15ml) and (2-Methyl-1H-indol-3-yl)(naphthalen-1-yl) methanone **2** (1.5g, 5.54mmol) was added portionwise at room temperature, allowing to stir further 30 min at room temperature after the addition was finished. Ethyl-4-bromoacetate (1.2ml, 8.31mmol) was added dropwise at room temperature and the reaction mixture was stirred at room temperature overnight. Solvents were removed in vacuo and the residue was partitioned between ethyl acetate (200ml) and water (150ml). The layers were separated and aqueous layer was extracted 2 times with ethyl acetate (2x100ml). All the organic fractions were combined,

dried over sodium sulphate and concentrated in vacuo. The residue was purified by column chromatography (silica gel, 20% ethyl acetate in hexane) to give 500mg (23%) of ethyl 4-(3-(1-naphthoyl)-2-methyl-1H-indol-1-yl) butanoate **3** as semi-solid.

**Example 31:** Preparation of 4-(3-(1-naphthoyl)-2-methyl-1H-indol-1-yl)butanoic acid (Hapten-E)

Ethyl 4-(3-(1-naphthoyl)-2-methyl-1H-indol-1-yl) butanoate **3** (500mg, 1.25mmol) was dissolved in tertahydrofuran (5ml) and water (5ml) was added followed by potassium hydroxide (206mg, 3.68mmol). The reaction mixture was stirred at room temperature overnight. The solvent was removed in vacuo, the residue was acidified to pH3 and extracted three times with a mixture 1:1 ethyl acetate:tetrahydrofuran (3x50ml). The organic fractions were combined, dried over sodium sulphate and concentrated under vacuo. The residue was purified by column chromatography (silica gel, ethyl acetate) to give 350mg (75%) 4-(3-(1-naphthoyl)-2-methyl-1H-indol-1-yl)butanoic acid **Hapten-E** as a yellow solid.

**Example 32:** Conjugation of 4-(3-(1-naphthoyl)-2-methyl-1H-indol-1-yl) butanoic acid (Hapten-E) to BSA (Immunogen-VII)

To a solution of 4-(3-(1-naphthoyl)-2-methyl-1H-indol-1-yl) butanoic acid **(Hapten-E)** (41.97mg, 0.09mM) in DMF (1.0ml) was added N, N-dicyclohexylcarbodiimide (DCC) (28.64mg, 0.12mM) and N-hydroxysuccinimide (14.3mg, 0.12mM) and the mixture was stirred at room temperature overnight. The dicyclohexylurea formed was removed by filtration and the solution was added drop-wise to a solution of BSA (150mg, 2.3μM) in 50mM sodium bicarbonate solution (pH 8.5) (10ml). The mixture was then stirred overnight at 4° C. The solution was then dialysed against 50mM phosphate buffer pH 7.2 (3 changes) for 24 hours at 4° C, and freeze-dried to give **Immunogen-VII**.

MALDI results showed 37.64 molecules of **hapten-E** had been conjugated to one molecule of BSA.

**Example 33:** Conjugation of 4-(3-(1-naphthoyl)-2-methyl-1H-indol-1-yl) butanoic acid (Hapten-E) to BTG (Immunogen-VII)

To a solution of 4-(3-(1-naphthoyl)-2-methyl-1H-indol-1-yl) butanoic acid **(Hapten-E)** (50.25mg, 0.14mM) in DMF (1.0ml) was added N, N-dicyclohexylcarbodiimide (DCC) (30.72mg, 0.15mM) and N-hydroxysuccinimide (17.13mg, 0.15mM) and the mixture was stirred at room temperature overnight. The dicyclohexylurea formed was removed by filtration and the solution was added drop wise to a solution of BTG (150mg) in 50mM sodium bicarbonate solution (pH 8.5) (10ml). The mixture was then stirred overnight at 4° C. The solution was then dialysed against 50mM phosphate buffer pH 7.2 (3 changes) for 24 hours at 4° C, and freeze-dried to give **immunogen-VII**.

**Example 34:** Conjugation of 4-(3-(1-naphthoyl)-2-methyl-1H-indol-1-yl) butanoic acid (Hapten - E) to HRP

EDC hydrochloride (10mg) was dissolved in water (0.5ml) and immediately added to a solution of 4-(3-(1-naphthoyl)-2-methyl-1H-indol-1-yl) butanoic acid **(Hapten-E)** (2mg) in DMF (0.2ml). After mixing, this solution was added drop wise to a solution of HRP (20mg) in water (1ml). Sulfo-NHS (5mg) was added and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight against 10L of PBS at pH 7.2 at 4° C.

**Preparation of Antisera**

In order to generate polyclonal antisera, each immunogen of the present invention is mixed with Freund's Adjuvant and the mixture is injected into a host animal, such as rabbit, sheep, mouse, guinea pig or horse. Sheep is the preferred host animal. Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate non-specific binding.

The specific antibodies prepared in this invention are useful as reagents in immunoassays for the detection or determination of synthetic cannabinoids and their metabolites in biological fluids.

**Example 35:** Preparation of antibodies to Immunogens I, II, IV, VII and VIII

***General method:***

An aqueous solution of each immunogen was formulated with Freund's Complete Adjuvant (FCA) to form an emulsion consisting of 2mg/ml* immunogen in 50% (v/v) FCA. Three sheep were immunised with this emulsion (1° immunisation), 0.25ml being intramuscularly injected at each of four sites in the flank of each animal. Subsequent immunizations (boosts) contained 1mg/ml* immunogen. All boosts were emulsified in 50% (v/v) Freund's Incomplete Adjuvant (FIA) and were administered in the same manner as the 1° immunisation, at monthly intervals. Blood sampling took place 7 to 14 days after each boost.
*The general procedure for immunogen administration is outlined above, however, it was necessary to deviate from this protocol in some cases, due to reduced immunogen concentrations (Immunogens II, IV, VII and VIII) and/or amount available (Immunogen VIII). Any deviations from the protocol described are outlined in Table 1A below and highlighted in bold.

**Table 1A:** Amount and volume of immunogens administered for primary and subsequent boosts

| Immunogen | Primary Amount/Volume | Boost Amount 25 /Volume |
|---|---|---|
| I | 2mg/1ml | 1mg/1ml |
| II | 2mg/**1.4ml** | 1mg/1ml |
| IV | 2mg/**1.3ml** | 1mg/1ml |
| VII | 2mg/**1.4ml** | 1mg/1ml |
| VIII | **1mg/1.45ml** | **0.5mg/1ml** |

*Blood collection*

Briefly, blood is collected by applying pressure to the exposed jugular vein and inserting a clean 14 gauge hypodermic needle to remove 500ml of blood per sheep, under gravity. The blood is stored at 37°C for a minimum of 1 hour before the clots are separated from the side of the centrifuge bottles using disposable 1ml pipettes (ringing). The samples are stored at 4°C overnight.

*Processing & extraction of Immunoglobulin (Ig) fraction:*

Samples are centrifuged at 4000g for 30 minutes at 4°C. The serum is then poured off and centrifuged again at 16,000g for 15 minutes at 4°C, before being aliquoted and stored at <-20°C.

Precipitation of IgG from polyclonal antisera is carried out in two steps, using caprylic acid initially to precipitate most of the non-Ig G proteins, including albumin, followed by ammonium sulphate to extract IgG from the supernatant. This method produces a highly purified IgG fraction.

8ml of 60mM sodium acetate buffer, pH 4.4 is added to 2ml of antisera, followed by the addition of 200μl of caprylic acid. The resulting mixture is mixed on a roller for 30 minutes at room temperature. The precipitate is removed by centrifuging the samples at 1000g for 20 minutes at 4°C and filtering the supernatant through a 0.2μm Acrodisc™ filter. 1.4ml of 0.5M carbonate-bicarbonate buffer, pH 10.7, is added to each sample supernatant and cooled to 4°C. 9ml of saturated ammonium sulphate solution is added slowly whilst shaking and the resulting mixture is placed on a roller for 30 minutes at room temperature. The precipitate is extracted by centrifuging the samples at 1000g for 35 minutes at 4°C. The supernatant is poured off and the pellet re-suspended in 2ml PBS, pH 7.2. The sample is dialysed overnight at 4°C in PBS, pH7.2 containing 0.09% azide. After dialysis, the sample is filtered using a 0.2μm Acrodisc™ filter and aliquoted for storage at <20°C. The IgG fraction can then be evaluated by competitive ELISA microtiter plate assay, as described below.

**Example 36:** Charaterisation of antibodies to Immunogens I, II, IV, VII and VIII

**Standard curves**

General method: Immunogen VI

The wells of an enhanced binding 96 well polystyrene microtiter plate were coated with IgG fraction of antiserum raised to Immunogen I or II, diluted in 10mM Tris, pH8.5 (125μl/well). The appropriate antibody coating dilution was determined using standard ELISA checkerboard techniques. The plate was incubated overnight at 4°C, washed 4 times over 10 minutes with Tris buffered saline containing Tween 20 (TBST) and tapped dry. Standard solutions (JWH-018 for Immunogens I and II) were applied at 0, 0.625, 1.25, 2.5, 5, 10, 20 and 40ng/ml and 50μl of each was added to the appropriate wells. 75μl of conjugate (appropriate hapten-HRP) diluted in Tris buffer (pH 7.2) containing EDTA, D-mannitol, sucrose, thimerosal and BSA, was added to each of the wells. The appropriate dilution of conjugate was also determined using standard ELISA checkerboard techniques. The plate was incubated at 25°C for 1 hour. Excess unbound conjugate was removed by washing 6 times over a 10 minute period with TBST. 125μl of tetramethylbenzidine (TMB) substrate solution was added to each well of the plate that was then incubated for 20 minutes in the dark at room temperature. The reaction was terminated by addition of 125μl 0.2M $H_2SO_4$ to each well. The absorbance was then measured at 450nm using a microtiter plate reader. The data are inputted to a computer program called 'KC Junior'. It is a 4 parameter fit curve and allow the calculatation of concentrations between the standard runs. This program is used to calculate the IC50s by dividing the 0ng/ml OD value by 2 and obtaining the concentration value from the curve for this OD. The data generated in the assay and inputted to a computer program called 'KC Junior' are presented in **Table 3**.

General method: Immunogens I and II

In a similar manner to that described above, the wells of a 96-well microtiter plate were coated with the IgG fraction of the antiserum raised to Immunogens I or II. Standard solutions (JWH-018 for Immunogen I and II) were applied at 0, 0.625, 1.25, 2.5, 5, 10, 20 and 40ng/ml and conjugate was employed as detection reagent. The data generated are presented in **Table 3.**

General method: Immunogen IV, VII, VIII

In a similar manner to that described above, the wells of a 96-well microtiter plate were coated with the IgG fraction of the antiserum raised to Immunogens IV, VII or VIII. Standard solutions (JWH-018 for Immunogens IV and VIII, JWH-015 for Immunogen VII) were prepared in TBST at 0, 1.25, 2.5, 5, 10, 20, 40 and 80ng/ml and conjugate was employed as detection reagent. The data generated are presented in **Table 3**.

The data set our in Tables 3 and 4 are generated using antibodies raised against the indicated Immunogens in a competitive binding assay with the indicated tracers, using the indicated standards:

**Table 2**

| | Immunogen/Hapten | Antibody | Standard | Tracer/hapten |
|---|---|---|---|---|
| Immunogen 1 | CJ-5-68/LK955 | RS1803B12 | JWH-018 | ESC4962/LK962 |
| Immunogen 2 | CJ-5-56/LK959 | RS1833B12 | JWH-018 | ESC4555/LK959 |
| Immunogen 4 | CJ-7-04/LK1122 | RS2142B5 | JWH-018 | ESC5913/LK1122 |
| Immunogen 7 | CJ-7-178/LK1199 | RS2255B1 | JWH-015 | ESC6616/LK1199 |
| Immunogen 8 | CJ-6-45/LK1037 | RS2099B5 | JWH-018 | ESC5794/LK1108 |

The tracers are illustrated in Figure 13 and the Immunogens in Figure 14. **Table 3:** Data generated from competitive microtiter plate assays for Synthetic Cannabinoids and their metabolites, employing antisera generated to Immunogens I, II, IV, VII and VIII

**Table 3a**

| JWH-018 | Antibody to Immunogen 1 | | JWH-018 | Antibody to Immunogen 2 | |
|---|---|---|---|---|---|
| ng/ml | $A_{450}$ | $\%B/B_0$ | ng/ml | A450 | %B/B0 |
| 0 | 1.61 | 100 | 0 | 1.4575 | 100 |
| 0.625 | 1.247 | 77 | 0.625 | 0.984 | 68 |
| 1.25 | 1.004 | 62 | 1.25 | 0.7685 | 53 |
| 2.5 | 0.707 | 44 | 2.5 | 0.499 | 34 |
| 5 | 0.504 | 31 | 5 | 0.3615 | 25 |
| 10 | 0.323 | 20 | 10 | 0.2525 | 17 |
| 20 | 0.22 | 14 | 20 | 0.1855 | 13 |
| 40 | 0.15 | 9 | 40 | 0.149 | 10 |
| $IC_{50}$ (ng/ml) | **2.016** | | $IC_{50}$ (ng/ml) | **1.333** | |

**Table 3b**

| JWH-018 | Antibody to Immunogen 4 | |
|---|---|---|
| ng/ml | A450 | %B/B0 |
| 0 | 1.6985 | 100 |
| 1.25 | 1.479 | 87 |
| 2.5 | 1.1625 | 68 |
| 5 | 0.7155 | 42 |
| 10 | 0.442 | 26 |
| 20 | 0.2645 | 16 |
| 40 | 0.1735 | 10 |
| 80 | 0.116 | 7 |
| IC50 (ng/ml) | **4.133** | |

**Table 3c**

| JWH-015 | Antibody to Immunogen 7 | | JWH-018 | Antibody to Immunogen 8 | |
|---|---|---|---|---|---|
| ng/ml | A450 | %B/B0 | ng/ml | A450 | %B/B0 |
| 0 | 2.035 | 100 | 0 | 1.3825 | 100 |
| 1.25 | 1.53 | 75 | 1 | 1.09 | 79 |
| 2.5 | 1.2605 | 62 | 3 | 0.8765 | 63 |
| 5 | 1.0875 | 54 | 5 | 0.708 | 51 |
| 10 | 0.869 | 43 | 10 | 0.5515 | 40 |
| 20 | 0.69 | 34 | 20 | 0.4015 | 29 |
| 40 | 0.5345 | 26 | 40 | 0.3095 | 22 |
| 80 | 0.407 | 20 | 80 | 0.217 | 16 |
| IC50 (ng/ml) | **5.910** | | IC50 (ng/ml) | **5.230** | |

$A_{450}$ = absorbance at 450nm

B = absorbance at 450nm at xng/ml standard concentration

$B_0$ = absorbance at 450nm at 0ng/ml standard concentration

$IC_{50}$ = standard concentration which produces 50% $B/B_0$

**Cross reactivity**

In order to determine the specificity of the competitive ELISAs, standard solutions of a range of structurally similar synthetic cannabinoids and their metabolites were prepared in TBST. Using the calibration curves generated and employing a single level of cross reactants, these were used to determine the cross-reactivity of each immunoassay with these substances. The results of this study are presented in **Table 4**, as $\%B/B_0$. These are the standards used for the $IC_{50}$ and cross-reactivity data generation

Immunogen I - JWH-018
Immunogen II - JWH-018
Immunogen IV - JWH-018
Immunogen VII - JWH-015
Immunogen VIII - JWH-018

**Table 4:** Cross reactivity of the competitive ELISAs for synthetic cannabinoids and their metabolites

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 1 | JWH-018 | 5.19 | 9.29 | 6.80 | 67.66 | 17.66 |
| 2 | 2-OH JWH-018 (JWH-018 2-hydroxyindole metabolite) | 95.37 | | 100.14 | 95.08 | 101.36 |
| 3 | 4-OH JWH-018 (JWH-018 4-hydroxyindole metabolite) | 46.39 | 29.49 | 20.42 | 89.96 | 16.72 |
| 4 | 5-OH JWH-018 (JWH-018 5-hydroxyindole metabolite) | 11.79 | 12.14 | 78.56 | 85.76 | 87.67 |
| 5 | 6-OH JWH-018 (JWH-018 6-hydroxyindole metabolite) | 8.07 | 24.39 | 51.00 | 68.17 | 89.86 |
| 6 | 7-OH JWH-018 (JWH-018 7-hydroxyindole metabolite) | 6.72 | 34.79 | 93.31 | 68.53 | 94.46 |
| 7 | N-desalkyl JWH-018: LK1012 10CD194 | 15.63 | 99.18 | 95.55 | 80.08 | 90.49 |
| 8 | (±)-JWH 018 N-(4-hydroxypentyl) metabolite | 4.23 | 53.99 | 52.29 | 57.37 | 21.94 |
| 9 | JWH-018 N-(5-hydroxypentyl) metabolite | 3.50 | 53.38 | 78.42 | 53.32 | 57.37 |
| 10 | JWH-018 N-pentanoic acid metabolite | 4.51 | 85.61 | 99.89 | 53.02 | 100.31 |
| 11 | JWH 018 N-(1,2-dimethylpropyl) isomer | 6.60 | 71.33 | 87.11 | 62.29 | 67.71 |
| 12 | JWH 018 2'-naphthyl isomer | 75.90 | | 28.13 | 94.98 | 14.94 |
| 13 | JWH 018 adamantyl analog | 95.94 | | 99.02 | 94.48 | 10.97 |
| 14 | JWH-018 N-(1-methylbutyl) isomer | 12.42 | 51.92 | 73.49 | 67.71 | 63.22 |
| 15 | JWH-018 N-(2,2-dimethylpropyl) isomer | 3.89 | 60.15 | 80.42 | 52.46 | 95.09 |

(continued)

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 16 | JWH-018 6-methoxyindole analogue | 10.55 | 20.44 | 90.65 | 72.33 | 77.22 |
| 17 | JWH-018 N-(2-methylbutyl) isomer (JWH-073 2-methylbutyl homologue) | 3.61 | 28.60 | 29.88 | 58.84 | 85.16 |
| 18 | JWH 018 N-(3-methylbutyl) isomer (JWH-073 3-methylbutyl homologue) | 3.16 | 30.38 | 12.75 | 48.81 | 53.29 |
| 19 | JWH-073 | 3.56 | 15.24 | 13.63 | 58.34 | 25.50 |
| 20 | 2-OH JWH-073 (JWH-073 2-hydroxyindole metabolite) | 89.62 | | 89.63 | 92.65 | 93.42 |
| 21 | 4-OH JWH-073 (JWH-073 4-hydroxyindole metabolite) | 39.50 | 40.14 | 28.69 | 82.82 | 26.23 |
| 22 | 5-OH JWH-073 (JWH-073 5-hydroxyindole metabolite) | 7.34 | 20.73 | 84.06 | 75.11 | 90.70 |
| 23 | 6-OH JWH-073 (JWH-073 6-hydroxyindole metabolite) | 5.93 | 28.03 | 74.89 | 61.83 | 93.52 |
| 24 | 7-OH JWH-073 (JWH-073 7-hydroxyindole metabolite) | 5.59 | 46.05 | 96.95 | 61.78 | 94.04 |
| 25 | JWH-073 N-(3-hydroxybutyl) metabolite | 3.10 | 54.52 | 53.52 | 43.39 | 94.04 |
| 26 | JWH-073 N-(4-hydroxybutyl) metabolite | 3.22 | 60.01 | 52.61 | 48.81 | 57.99 |
| 27 | JWH-073 N-butanoic acid metabolite | 6.88 | 92.20 | 98.95 | 60.01 | 94.98 |
| 28 | JWH 073 2-methylnaphthyl analog | 21.84 | 43.34 | 34.47 | 76.74 | 78.79 |

(continued)

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 29 | JWH-073 4-methylnaphthyl analogue | 45.54 | | 7.01 | 78.36 | 19.75 |
| 30 | JWH-007 | 32.96 | 66.06 | 60.63 | 27.77 | 61.76 |
| 31 | JWH-011 | 62.13 | | 99.19 | 57.43 | 97.49 |
| 32 | JWH-015 | 14.67 | 65.67 | 93.20 | 17.84 | 92.27 |
| 33 | JWH-016 | 20.71 | 68.73 | 91.14 | 19.31 | 75.24 |
| 34 | JWH-019 | 7.22 | 15.42 | 21.96 | 70.05 | 74.50 |
| 35 | JWH 019 5-hydroxyindole metabolite (JWH-019-M2) | 19.81 | 17.34 | 93.84 | 87.58 | 101.57 |
| 36 | JWH-020 | 16.03 | 35.75 | 73.73 | 81.30 | 100.73 |
| 37 | JWH-022 | 4.51 | 11.72 | 10.61 | 58.44 | 28.94 |
| 38 | JWH-030 | 61.17 | | 93.17 | 89.66 | 94.25 |
| 39 | JWH-081 | 62.13 | | 3.78 | 86.72 | 15.88 |
| 40 | JWH-081 2-methoxynaphthyl isomer or (JWH-267) | 69.58 | | 27.67 | 83.43 | 58.20 |
| 41 | JWH-081 5-methoxynaphthyl isomer | 18.51 | 60.90 | 6.24 | 94.16 | 26.56 |
| 42 | YWH-081 7-methoxynaphthyl isomer (JWH-164) | 88.26 | | 39.20 | 97.60 | 59.51 |
| 43 | JWH-081 N-(5-hydroxypentyl) metabolite | 61.86 | | 26.31 | 89.31 | 47.16 |
| 44 | JWH-098 | 77.58 | | 44.03 | 82.85 | 59.80 |
| 45 | JWH-122 | 62.98 | 44.55 | 3.94 | 89.37 | 14.43 |
| 46 | JWH 122 6-methylnaphthyl isomer | 12.55 | 39.17 | 8.12 | 86.66 | 20.46 |
| 47 | JWH 122 7-methylnaphthyl isomer | 36.46 | 38.28 | 13.48 | 92.44 | 38.48 |

(continued)

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 48 | JWH 122 2-methylnaphthyl isomer | 33.35 | 27.14 | 26.13 | 85.37 | 61.74 |
| 49 | JWH-122 N-(5-hydroxypentyl) metabolite | 42.80 | 71.76 | 25.72 | 83.65 | 48.67 |
| 50 | JWH-133 | 100.56 | | 98.18 | 101.84 | 100.00 |
| 51 | JWH-147 | 83.85 | | 97.00 | 99.32 | 92.32 |
| 52 | JWH-164 (JWH-081 7-methoxynaphthyl isomer) | 91.86 | | 36.14 | 96.13 | 57.93 |
| 53 | JWH-182 | 107.08 | | 12.83 | 98.40 | 30.65 |
| 55 | JWH-200 4-hydroxyindole metabolite | 44.97 | 78.17 | 87.88 | 91.15 | 91.82 |
| 56 | JWH-200 5-hydroxyindole metabolite | 12.61 | 64.99 | 97.53 | 82.42 | 90.88 |
| 57 | JWH-200 6-hydroxyindole metabolite | 7.27 | 61.65 | 94.41 | 65.52 | 83.20 |
| 58 | JWH-200 2'-naphthyl isomer | 28.57 | | 92.29 | 94.65 | 96.12 |
| 59 | JWH-201 | 77.14 | | 59.03 | 100.25 | 8.26 |
| 60 | JWH-203 | 72.55 | | 52.15 | 99.08 | 9.19 |
| 61 | JWH 203 3-chloro isomer (JWH-237) | 95.59 | | 55.09 | 97.42 | 10.34 |
| 62 | JWH-206 (JWH-203 4-chloro isomer) | 75.96 | | 65.63 | 97.17 | 10.55 |
| 63 | JWH-210 | 91.18 | | 5.53 | 96.62 | 16.87 |
| 64 | JWH-210 2-ethylnaphthyl isomer | 81.37 | | 53.97 | 96.13 | 73.51 |
| 65 | JWH-210 7-ethylnaphthyl isomer or JWH-234 | 73.11 | | 27.07 | 100.55 | 50.90 |

(continued)

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 66 | JWH-210 N-(5-carboxypentyl) metabolite | 76.77 | | 85.29 | 96.07 | 96.27 |
| 67 | JWH-210 5-hydroxyindole metabolite | 83.29 | | 48.91 | 100.43 | 87.72 |
| 68 | JWH-250 | 78.94 | | 41.08 | 97.23 | 9.40 |
| 69 | JWH 250 N-(5-hydroxypentyl) metabolite | 75.47 | | 76.22 | 96.13 | 42.79 |
| 70 | JWH 250 N-(5-carboxypentyl) metabolite | 86.21 | | 89.88 | 97.60 | 89.30 |
| 71 | JWH 250 5-hydroxyindole metabolite | 100.75 | | 84.05 | 99.88 | 67.19 |
| 72 | JWH-251 | 68.01 | | 42.02 | 96.74 | 9.12 |
| 73 | JWH 251 3-methylphenyl isomer | 85.90 | | 51.15 | 99.32 | 8.97 |
| 74 | JWH-302 | 84.66 | | 45.67 | 104.92 | 7.97 |
| 75 | JWH-398 | 60.31 | 30.31 | 6.00 | 94.47 | 18.66 |
| 76 | JWH-398 5-chloronaphthyl isomer | 35.53 | 23.50 | 7.06 | 91.33 | 19.67 |
| 77 | JWH-398 N-(5-hydroxypentyl) metabolite | 48.07 | 67.49 | 22.01 | 82.97 | 51.33 |
| 78 | AM-630 (other name 6-Iodopravadoline) | 91.12 | | 90.17 | 99.88 | 89.88 |
| 79 | AM-694 | 12.17 | 36.18 | 11.30 | 75.66 | 11.92 |
| 80 | AM-694 3-iodo isomer | 31.37 | 48.58 | 37.20 | 89.49 | 12.71 |
| 81 | AM-694 4-iodo isomer | 73.79 | | 27.66 | 98.71 | 13.42 |
| 82 | AM-1220 | 4.45 | 77.46 | 86.93 | 49.71 | 106.65 |
| 84 | AM-1241 | 85.61 | | 91.38 | 94.96 | 102.46 |

(continued)

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 85 | AM-2201 | 3.72 | 11.72 | 6.20 | 55.28 | 16.74 |
| 86 | AM-2201 N-(4-fluoropentyl) isomer | 3.45 | 14.60 | 5.72 | 52.70 | 15.00 |
| 87 | AM-2201 N-(4-hydroxypentyl) metabolite | 3.80 | 46.55 | 43.62 | 52.75 | 36.41 |
| 88 | AM-2233 | 16.97 | 86.43 | 92.10 | 72.46 | 91.25 |
| 89 | CP-49,497-C7 ((+-) CP 47,497) | 101.20 | | 96.84 | 94.23 | 86.95 |
| 90 | CP-47,497-para-quinone analogue | 100.26 | | 97.33 | 93.29 | 88.11 |
| 91 | CP-49,497-C8-homologue ((+- CP) 47,497-C8-homologue) | 101.11 | | 94.66 | 98.55 | 104.88 |
| 92 | ((+-)-CP 55,940) | 100.61 | | 94.60 | 100.25 | 100.25 |
| 93 | (-)-CP 55,940 | 98.27 | | 93.02 | 101.15 | 97.58 |
| 94 | (+)-CP 55,940 | 103.25 | | 91.38 | 97.15 | 95.12 |
| 95 | HU-210 | 102.57 | | 92.53 | 100.39 | 93.42 |
| 96 | HU-211 (Dexanabinol) | 103.36 | | 93.37 | 96.78 | 92.70 |
| 97 | HU-308 | 101.35 | | 94.03 | 95.95 | 89.77 |
| 98 | RCS-4 | 80.46 | | 19.21 | 93.51 | 5.93 |
| 99 | RCS-4 2-methoxy isomer | 29.14 | 42.74 | 15.28 | 87.27 | 29.32 |
| 100 | RCS-4 3-methoxy isomer | 31.19 | | 25.65 | 94.72 | 10.95 |
| 101 | RCS-4-C4 homologue (BTM-4, SR-19, OBT-199, E-4) | 76.27 | | 27.17 | 100.20 | 9.54 |
| 102 | RCS-4 N-(4-hydroxypentyl) metabolite | 65.71 | | 59.16 | 95.72 | 8.89 |
| 103 | RCS-4 N-(5-hydroxypentyl) metabolite | 59.51 | | 61.03 | 94.55 | 19.13 |

(continued)

| Cross-reactants at 100 ng/ml; *100ng/ml* | | Antibody to Immunogen I | Antibody to Immunogen II | Antibody to Immunogen IV | Antibody to Immunogen VII | Antibody to Immunogen VIII |
|---|---|---|---|---|---|---|
| | | %B/B0 | %B/B0 | %B/B0 | %B/B0 | %B/B0 |
| 104 | RCS-4 N-(5-carboxypentyl) metabolite | 77.12 | | 91.44 | 94.00 | 91.97 |
| 105 | RCS-8 (SR-18) | 82.65 | | 85.50 | 94.57 | 88.79 |
| 106 | RCS-8 3-methoxy isomer | 93.24 | | 89.43 | 93.73 | 83.62 |
| 107 | RCS-8 4-methoxy isomer | 94.94 | | 90.78 | 98.21 | 100.72 |
| 108 | (+)WIN 55212-2 (mesylate) | 17.00 | 83.87 | 92.76 | 24.08 | 107.12 |
| 109 | Win 55,212-3 mesylate | 34.00 | | 94.57 | 32.31 | 101.30 |
| 110 | WIN-48,098 (other name Pravadoline) | 90.05 | | 91.64 | 92.41 | 97.90 |
| 111 | WIN 55,225 (other name JWH-200) | 3.51 | 54.02 | 77.08 | 42.85 | 98.81 |
| 112 | *Delta 9 THC* | *102 66* | | 93.62 | 96.76 | 96.10 |
| 113 | *Indole-3-carboxylic Acid* | *101 26* | | 93.42 | 96.90 | 96.28 |
| 114 | *Cannabinol* | *86 60* | | 94.74 | 95.43 | 94.03 |
| 115 | *5-hydroxyindole-3-acetic acid (5-HIAA)* | *98 71* | | 96.61 | 99.46 | 108.24 |
| 116 | *Serotonin HCl* | *99 65* | | 95.40 | 99.83 | 100.36 |
| 117 | *5-hydroxytryptophol* | *93 30* | | 94.51 | 98.45 | 102.93 |
| 118 | *(-)-11-nor-9-Carboxy-delta9-THC* | *100 47* | | 93.08 | 98.72 | 103.36 |

| Cross-Reactant | CAS Registry Number |
|---|---|
| JWH-018 | 209414-07-3 |
| 2-OH JWH-018 (JWH-018 2-hydroxyindole metabolite) | |
| 4-OH JWH-018 (JWH-018 4-hydroxyindole metabolite) | |
| 5-OH JWH-018 (JWH-018 5-hydroxyindole metabolite) | 335161-21-2 |
| 6-OH JWH-018 (JWH-018 6-hydroxyindole metabolite) | |
| 7-OH JWH-018 (JWH-018 7-hydroxyindole metabolite) | |
| N-desalkyl JWH-018: LK1012 10CD194 | |
| (±)-JWH 018 N-(4-hydroxypentyl) metabolite | |
| JWH-018 N-(5-hydroxypentyl) metabolite | |
| JWH-018 N-pentanoic acid metabolite | |
| JWH 018 N-(1,2-dimethylpropyl) isomer | |
| JWH 018 2'-naphthyl isomer | 1131605-25-8 |
| JWH 018 adamantyl analog | |
| JWH-018 N-(1-methylbutyl) isomer | |
| JWH-018 N-(2,2-dimethylpropyl) isomer | |
| JWH-018 6-methoxyindole analogue | |
| JWH-018 N-(2-methylbutyl) isomer (JWH-073 2-methylbutyl homologue) | |
| JWH 018 N-(3-methylbutyl) isomer (JWH-073 3-methylbutyl homologue) | |
| JWH-073 | 208987-48-8 |
| 2-OH JWH-073 (JWH-073 2-hydroxyindole metabolite) | |
| 4-OH JWH-073 (JWH-073 4-hydroxyindole metabolite) | |
| 5-OH JWH-073 (JWH-073 5-hydroxyindole metabolite) | |
| 6-OH JWH-073 (JWH-073 6-hydroxyindole metabolite) | |
| 7-OH JWH-073 (JWH-073 7-hydroxyindole metabolite) | |
| JWH-073 N-(3-hydroxybutyl) metabolite | |
| JWH-073 N-(4-hydroxybutyl) metabolite | |
| JWH-073 N-butanoic acid metabolite | |
| JWH 073 2-methylnaphthyl analog | |
| JWH-073 4-methylnaphthyl analogue | |
| JWH-007 | 155471-10-6 |
| JWH-011 | 155471-13-9 |
| JWH-015 | 155471-08-2 |
| JWH-016 | 155471-09-3 |
| JWH-019 | 209414-08-4 |
| JWH 019 5-hydroxyindole metabolite (JWH-019-M2) | |
| JWH-020 | 209414-09-5 |
| JWH-022 | 209414-16-4 |
| JWH-030 | 162934-73-8 |

(continued)

| Cross-Reactant | CAS Registry Number |
|---|---|
| JWH-081 | 210179-46-7 |
| JWH-081 2-methoxynaphthyl isomer or (JWH-267) | 824960-76-1 |
| JWH-081 5-methoxynaphthyl isomer | |
| JWH-081 7-methoxynaphthyl isomer (JWH-164) | 824961-61-7 |
| JWH-081 N-(5-hydroxypentyl) metabolite | |
| JWH-098 | 316189-74-9 |
| JWH-122 | 619294-47-2 |
| JWH 122 6-methylnaphthyl isomer | |
| JWH 122 7-methylnaphthyl isomer | 824960-56-7 |
| JWH 122 2-methylnaphthyl isomer | |
| JWH-122 N-(5-hydroxypentyl) metabolite | |
| JWH-133 | 259869-55-1 |
| JWH-147 | 914458-20-1 |
| JWH-164 (JWH-081 7-methoxynaphthyl isomer) | 824961-61-7 |
| JWH-182 | 824960-02-3 |
| JWH-200 4-hydroxyindole metabolite | |
| JWH-200 5-hydroxyindole metabolite | |
| JWH-200 6-hydroxyindole metabolite | |
| JWH-200 2'-naphthyl isomer | 133438-66-1 |
| JWH-201 | 864445-47-6 |
| JWH-203 | 864445-54-5 |
| JWH 203 3-chloro isomer (JWH-237) | 864445-56-7 |
| JWH-206 (JWH-203 4-chloro isomer) | 864445-58-9 |
| JWH-210 | 824959-81-1 |
| JWH-210 2-ethylnaphthyl isomer | |
| JWH-210 7-ethylnaphthyl isomer or JWH-234 | 824960-64-7 |
| JWH-210 N-(5-carboxypentyl) metabolite | |
| JWH-210 5-hydroxyindole metabolite | |
| JWH-250 | 864445-43-2 |
| JWH 250 N-(5-hydroxypentyl) metabolite | |
| JWH 250 N-(5-carboxypentyl) metabolite | |
| JWH 250 5-hydroxyindole metabolite | |
| JWH-251 | 864445-39-6 |
| JWH 251 3-methylphenyl isomer | |
| JWH-302 | 864445-45-4 |
| JWH-398 | 1292765-18-4 |
| JWH-398 5-chloronaphthyl isomer | |
| JWH-398 N-(5-hydroxypentyl) metabolite | |

(continued)

| Cross-Reactant | CAS Registry Number |
|---|---|
| AM-630 (other name 6-Iodopravadoline) | 164178-33-0 |
| AM-694 | 335161-03-0 |
| AM-694 3-iodo isomer | |
| AM-694 4-iodo isomer | |
| AM-1220 | 137642-54-7 |
| AM-1241 | 444912-48-5 |
| AM-2201 | 335161-24-5 |
| AM-2201 N-(4-fluoropentyl) isomer | |
| AM-2201 N-(4-hydroxypentyl) metabolite | |
| AM-2233 | 444912-75-8 |
| CP-49,497-C7 ((+-) CP 47,497) | |
| CP-47,497-para-quinone analogue | |
| CP-49,497-C8-homologue ((+- CP) 47,497-C8-homologue) | |
| ((+-)-CP 55,940) | |
| (-)-CP 55,940 | |
| (+)-CP 55,940 | |
| HU-210 | |
| HU-211 (Dexanabinol) | |
| HU-308 | |
| RCS-4 | 1345966-78-0 |
| RCS-4 2-methoxy isomer | |
| RCS-4 3-methoxy isomer | |
| RCS-4-C4 homologue (BTM-4, SR-19, OBT-199, E-4) | |
| RCS-4 N-(4-hydroxypentyl) metabolite | |
| RCS-4 N-(5-hydroxypentyl) metabolite | |
| RCS-4 N-(5-carboxypentyl) metabolite | |
| RCS-8 (SR-18) | 1345970-42-4 |
| RCS-8 3-methoxy isomer | |
| RCS-8 4-methoxy isomer | |
| (+)WIN 55212-2 (mesylate) | 131543-23-2 |
| Win 55,212-3 mesylate | 131543-25-4 |
| WIN-48,098 (other name Pravadoline) | 92623-83-1 |
| WIN 55,225 (other name JWH-200) | 103610-04-4 |
| *Delta 9 THC* | 1972-08-3 |
| *Indole-3-carboxylic Acid* | 87-51-4 |
| *Cannabinol* | 521-35-7 |
| *5-hydroxyindole-3-acetic acid (5-HIAA)* | 54-16-0 |
| *Serotonin HCl* | 153-98-0 |

(continued)

| Cross-Reactant | CAS Registry Number |
|---|---|
| *5-hydroxytryptophol* | 154-02-9 |
| *(-)-11-nor-9-Carboxy-delta9-THC* | 56354-06-4 |

## Claims

**1.** An antibody raisable against one or more immunogens possessing the following structures

(a)

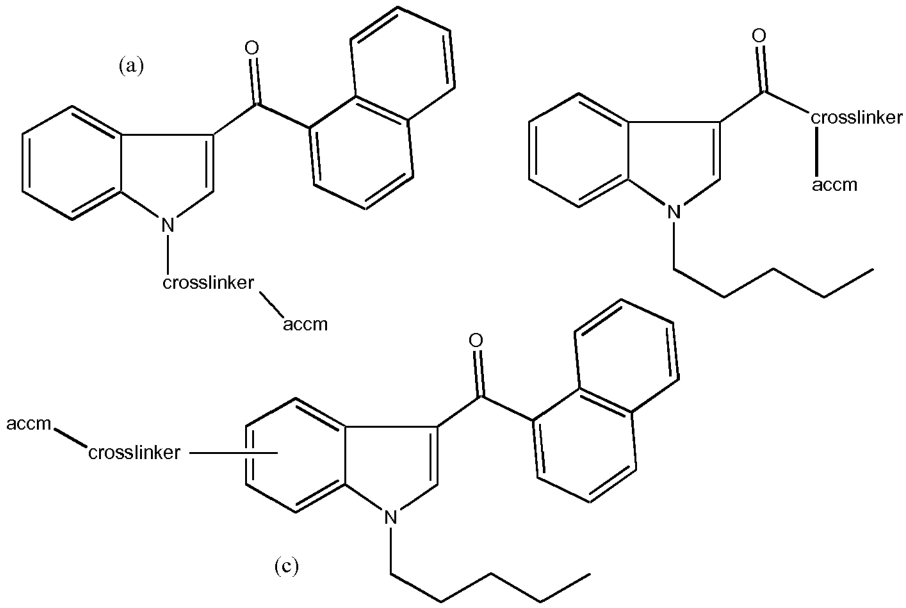

(b)

(c)

(d)

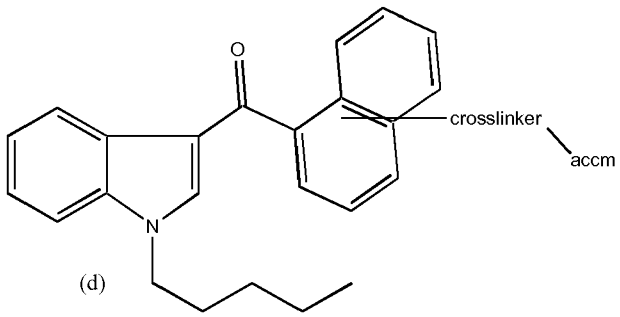

Group I (a)-(d) (immunogens of the JWH family)

in which the accm is an antigenicity conferring carrier material; the crosslinker is a functionalised linking group joining the accm to the remainder of the molecule and in which, in structure (a), the 2-position of the indole group can optionally be substituted with a methyl group.

**2.** The antibody of Claim 1 which binds to an epitope of structure

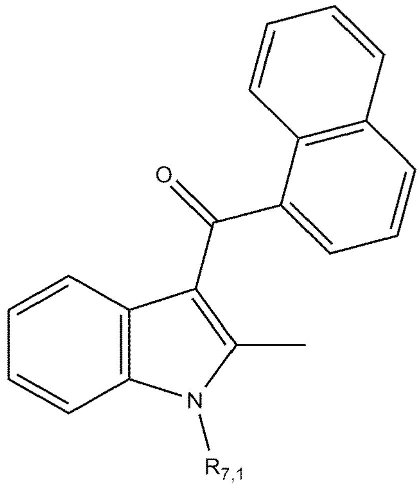

wherein,

$R_{7,1}$ is a $C_3$ or $C_4$ hydrocarbon chain.

3. The antibody of Claim 2 which binds to an epitope of the molecules JWH-015 and JWH-016.

4. The antibody of Claim 2 or 3 further **characterised by** being raisable from an immunogen of structure VII and / or further **characterised by** having a $B/B_0$ of ≤ 20% in Table 4 (standardised with RCS-4 and using tracer ESC6585).

5. The antibody of Claim 1 which binds to an epitope of structure

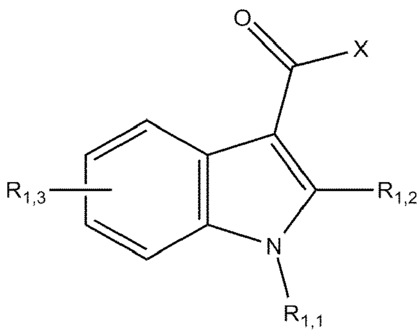

wherein,

$R_{1.1}$ is H or a $C_1$ to $C_6$, substituted or unsubstituted, saturated, hydrocarbon chain;
$R_{1,2}$ is H or methyl, optionally H;
$R_{1,3}$, if present, is methoxy or OH; and
X is a substituted or unsubstituted naphthyl moiety, optionally an unsubstituted naphthyl moiety, or a substituted benzyl moiety.

6. The antibody of Claim 5 in which $R_{1,1}$ is selected from H; substituted methyl, substituted ethyl, substituted or unsubstituted propyl, substituted or unsubstituted butyl, substituted or unsubstituted pentyl or hexyl; wherein $R_{1,1}$ is, optionally, selected from (1-methyl-2-piperidinyl)methyl, ethyl-morpholino, propyl, substituted propyl selected from 1,2-dimethylpropyl, 2,2-dimethylpropyl or butyryl; butyl, substituted butyl selected from valeryl, 1-methylbutyl, 2-metylbutyl, 3-methylbutyl, 3-hydroxybutyl, 4-hydroxybutyl, pentyl, substituted pentyl selected from 4-hydroxypentyl, 5-hydroxypentyl, 5-fluoropentyl, 4-fluoropentyl and 4-hydroxy-5-fluoropentyl, 4-pentenyl and hexyl and / or in which $R_{1,3}$, if present, is present at one of positions 5, 6 or 7 of the indole ring, optionally, at one of positions 6 or 7 of the indole ring.

7. The antibody of Claim 5 or 6 in which, when X is substituted naphthyl, the naphthyl is substituted at the 6-position

of the naphthyl ring with, optionally, methyl or, when X is substituted phenyl, the phenyl is substituted at position 2 of the phenyl ring with, optionally, iodine.

**8.** The antibody of any one of Claims 5 to 7 further **characterised by** being raisable from an immunogen of structure I and / or further **characterised by** having a $B/B_0$ of $\leq 16\%$ in Table 4 (standardised with JWH-018 and using tracer ESC6557).

**9.** The antibody of Claim 1 which binds to an epitope of structure

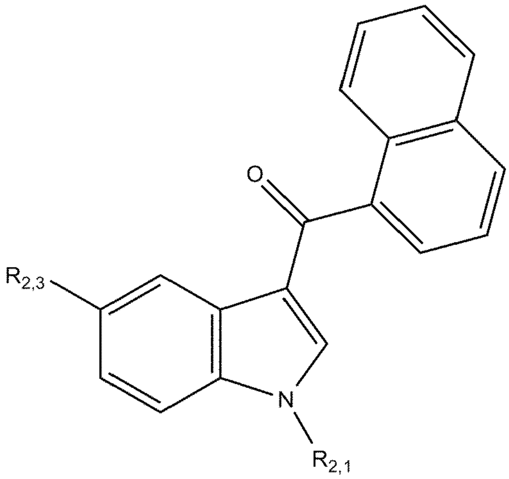

wherein,

$R_{2,1}$ is $C_4$ to $C_6$, substituted or unsubstituted, saturated or unsaturated, hydrocarbon chain; and
$R_{2,3}$ is H or hydroxyl.

**10.** The antibody of Claim 9 in which $R_{2,1}$ is selected from butyl, pentyl, substituted pentyl selected from 4-fluoropentyl and 5-fluoropentyl, 4-pentenyl and hexyl.

**11.** The antibody of Claims 9 or 10 further **characterised by** having been derived from an immunogen of structure II and / or further **characterised by** having a $B/B_0$ of $\leq 15\%$ in Table 4 (standardised with JWH-018 and using tracer ESC6557).

**12.** The antibody of Claim 1 which binds to an epitope of structure

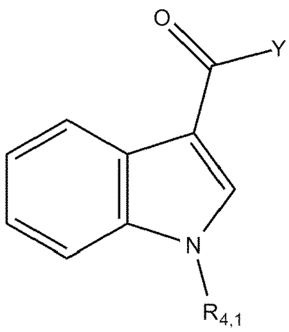

wherein,

$R_{4,1}$ is a $C_4$ or $C_5$, substituted or unsubstituted, saturated or unsaturated, hydrocarbon chain;
Y is substituted or unsubstituted naphthyl moiety or substituted phenyl moiety, in which Y is optionally a substituted naphthyl moiety;
in which, optionally, $R_{4,1}$ is selected from butyl, substituted butyl selected from 3-methylbutyl; pentyl; substituted

pentyl selected from 4-fluoropentyl and 5-fluoropentyl; and 4-pentenyl; and / or
in which, optionally, Y is substituted naphthyl and the naphthyl is substituted with methyl, ethyl, propyl, methoxy or chlorine, optionally at one of positions 4, 5, 6 or 7 of the naphthyl ring;
further optionally, in which Y is substituted phenyl, the phenyl is substituted with methoxy or iodine, optionally at position 2 of the phenyl ring and $R_{4,1}$ is selected from pentyl and 5-fluoropentyl; or
further optionally in which Y is substituted naphthyl and the naphthyl is substituted with methyl or methoxy at position 4 of the naphthyl ring and $R_{4,1}$ is pentyl.

**13.** The antibody of any one of Claims 9 to 12 further **characterised by** being raisable from an immunogen of structure IV and / or having a $B/B_0$ of ≤ 15% in Table 4 (standardised with RCS-8 and using tracer ESC6557).

**14.** The antibody of Claim 1 which binds to an epitope of structure

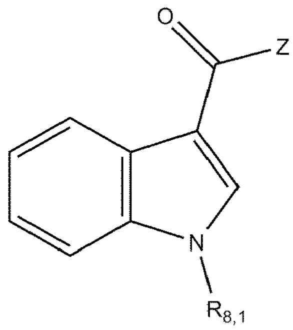

wherein,

$R_{8,1}$ is a $C_4$ or $C_5$, substituted or unsubstituted, hydrocarbon chain;
Z is substituted or unsubstituted 1- or 2-naphthyl, adamantyl, substituted benzyl or substituted phenyl moiety; in which, optionally, $R_{8,1}$ is selected from butyl; pentyl; and substituted pentyl, optionally selected from 5-fluoropentyl, 4-fluoropentyl and 4-hydroxypentyl; and / or
in which, when Z is substituted 1- or 2-naphthyl, the naphthyl is substituted with methyl or methoxy, optionally at the 4 position on the naphthyl ring or, when Z is substituted benzyl, the benzyl is substituted with methyl optionally at the ortho or meta positions on the phenyl ring, methoxy or chlorine or, when $R_3$ is substituted phenyl, the phenyl is substituted with methoxy optionally at the meta or para positions of the phenyl ring or iodine.

**15.** The antibody of Claim 14 further **characterised by** being raisable from an immunogen of structure VIII. and / or having a $B/B_0$ of ≤ 20% in Table 4 (standardised to JWH-018 and using tracer ESC5794).

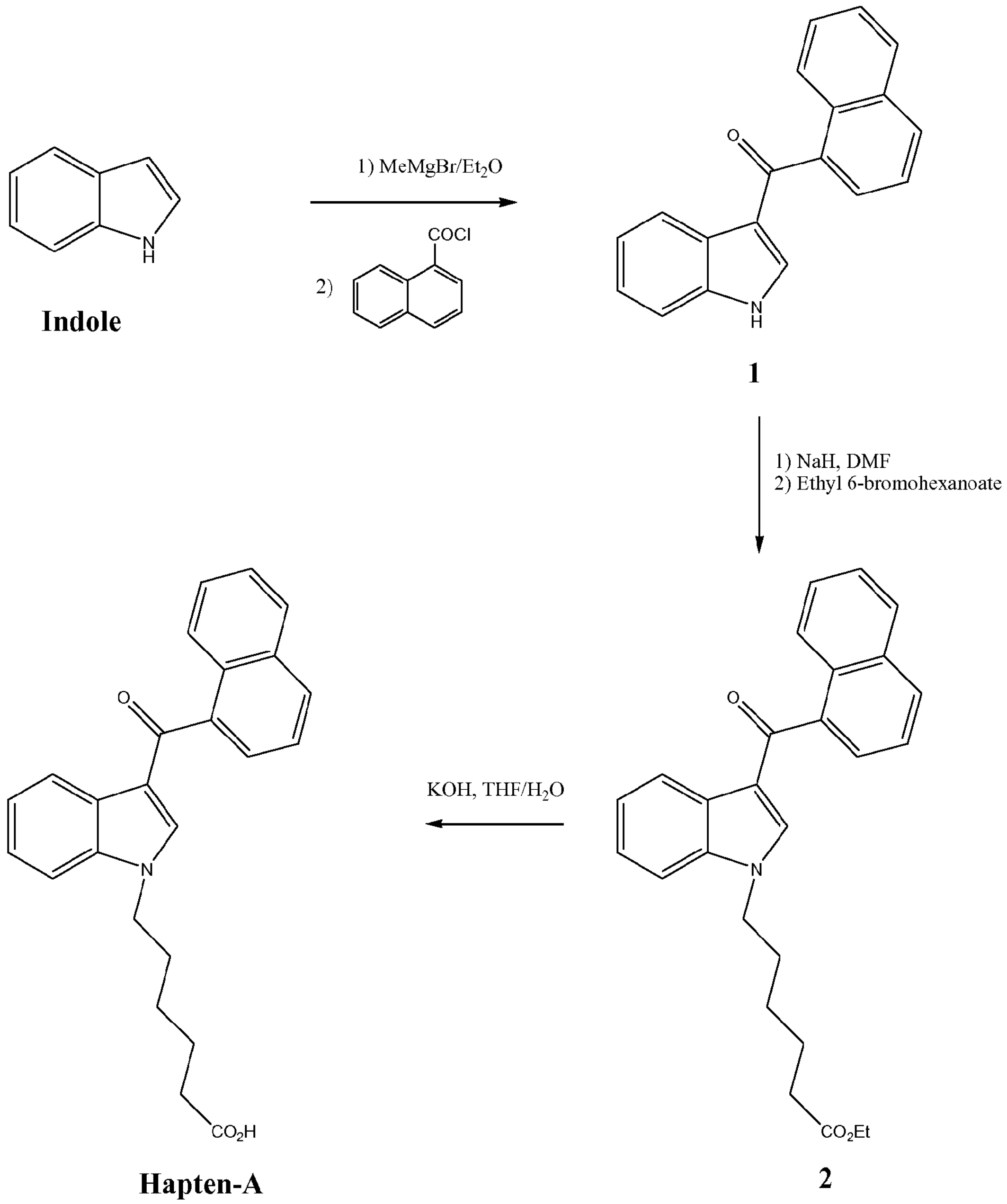


Indole

1

2

Hapten-A

**Figure 1**

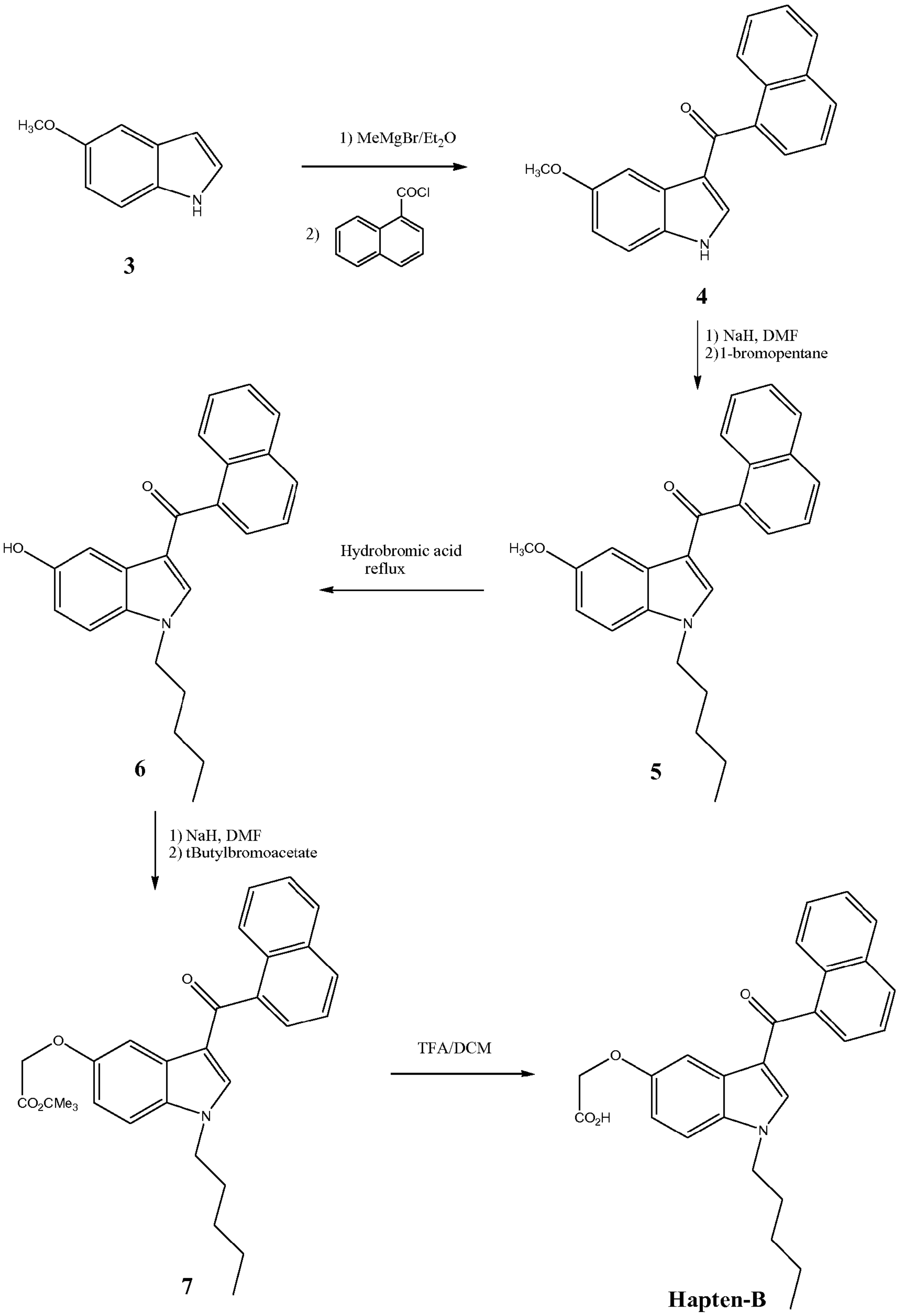
3
1) MeMgBr/Et$_2$O
2)
4
1) NaH, DMF
2) 1-bromopentane
Hydrobromic acid
reflux
6
5
1) NaH, DMF
2) tButylbromoacetate
TFA/DCM
7
Hapten-B

**Figure 2**

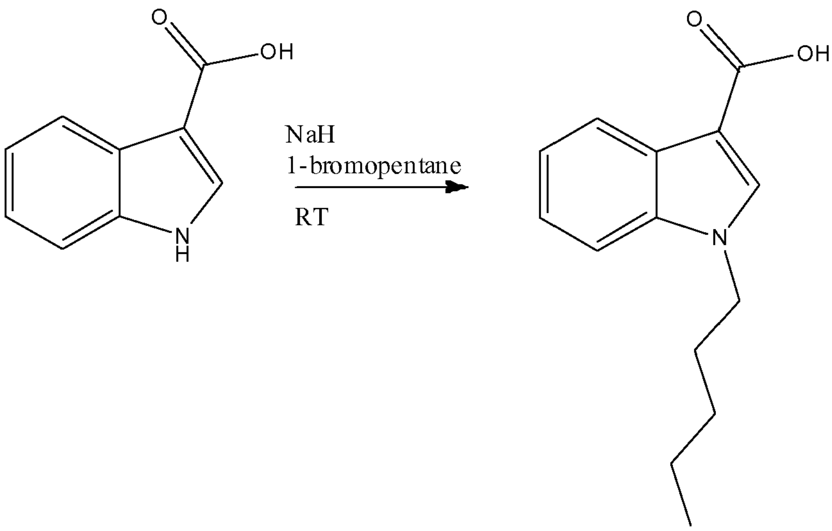


Hapten-C

**Figure 3**

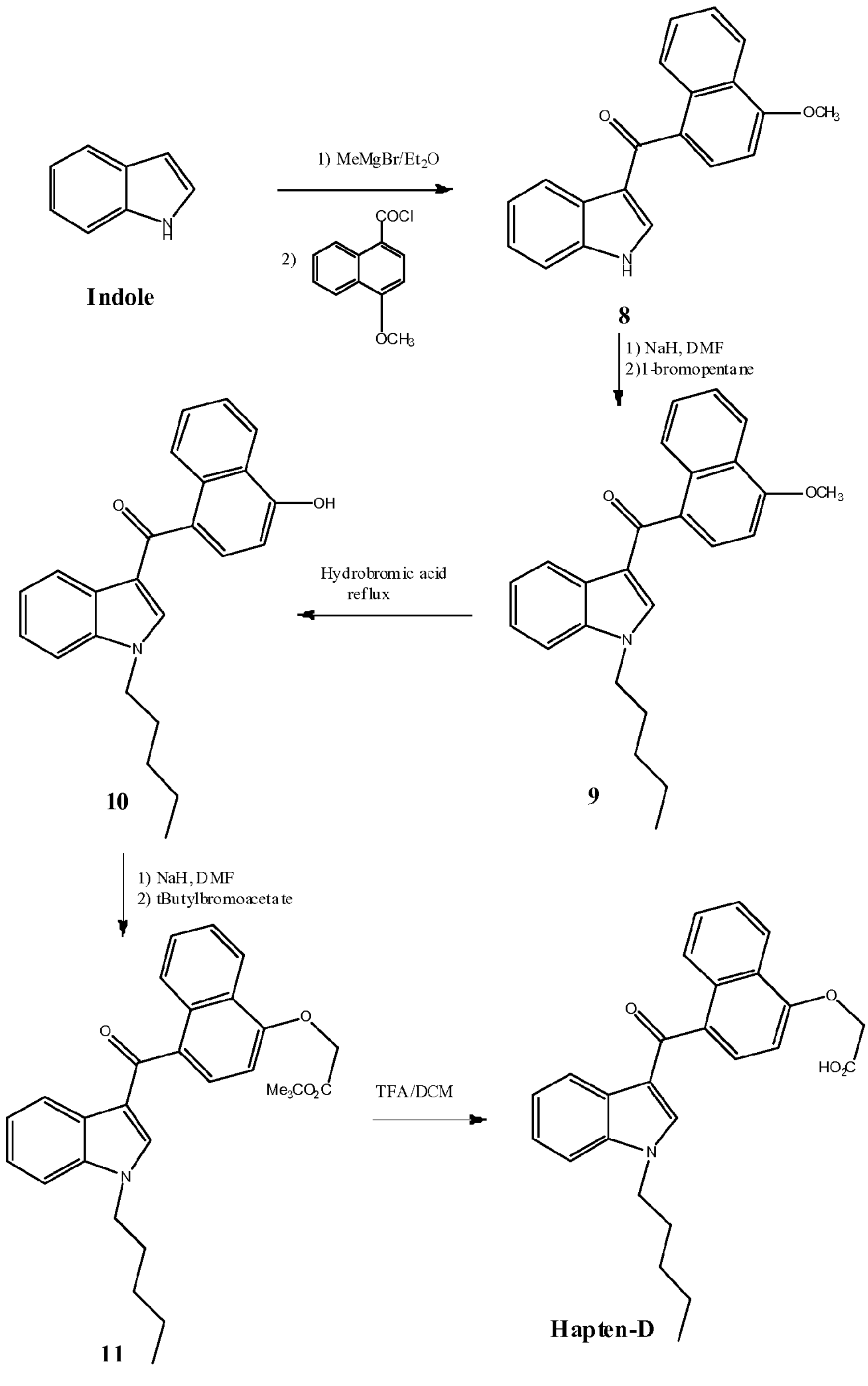


Indole

8

9

10

11

Hapten-D

**Figure 4**

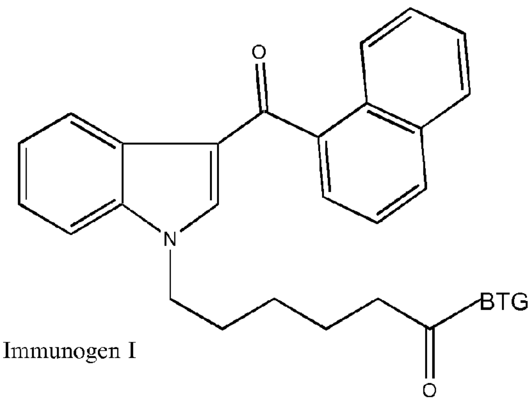

Immunogen I

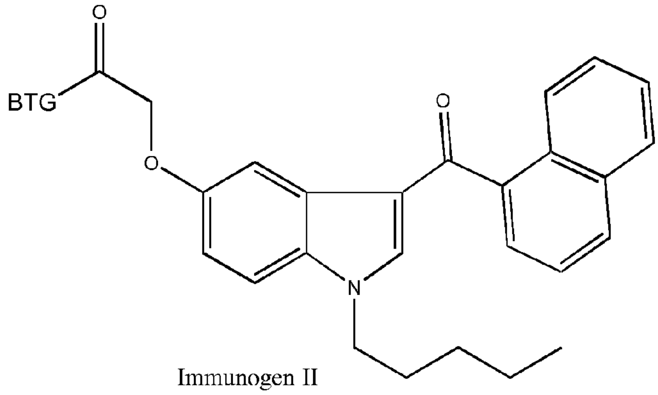

Immunogen II

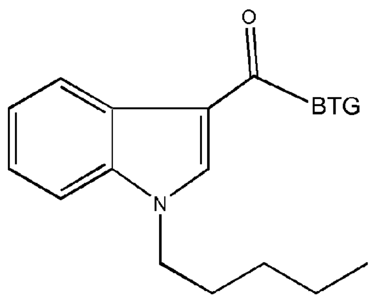

Immunogen III

**Figure 5 Immunogen III herein is now referred to as Immunogen VIII in the accompanying description**

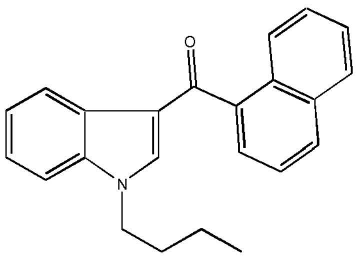

JWH-073

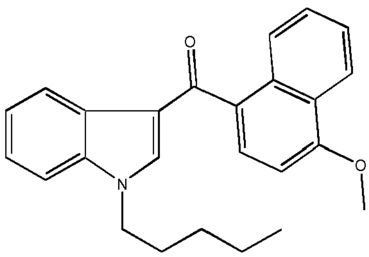

JWH-081

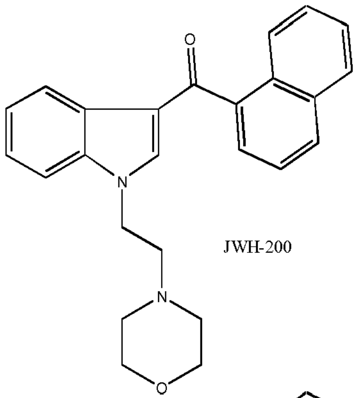

JWH-200

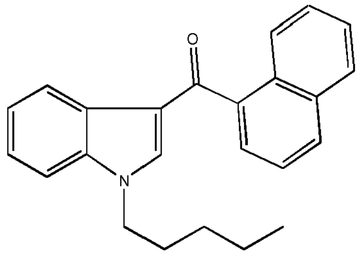

JWH-018

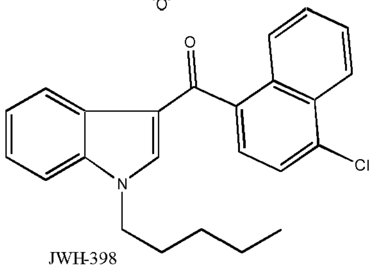

JWH-398

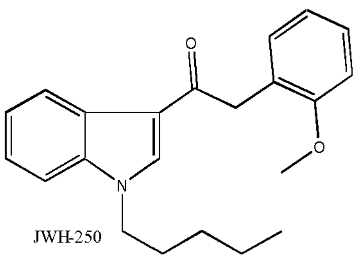

JWH-250

**Figure 6**

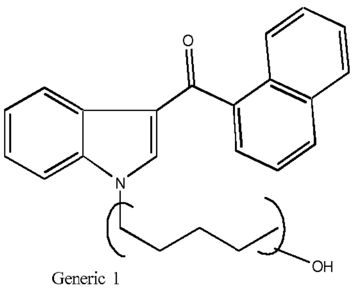

Generic 1

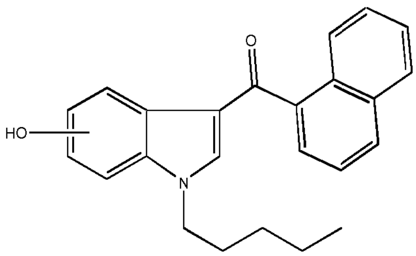

Generic 2

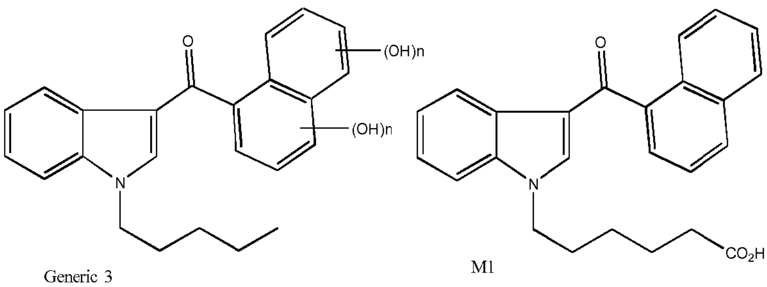

Generic 3

M1

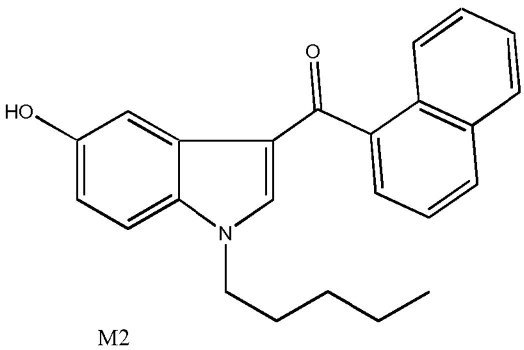

M2

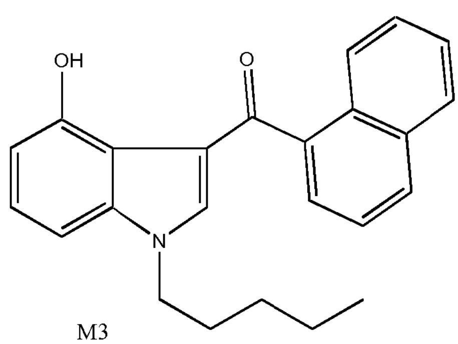

M3

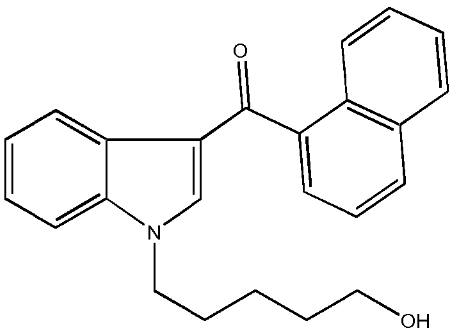

M4

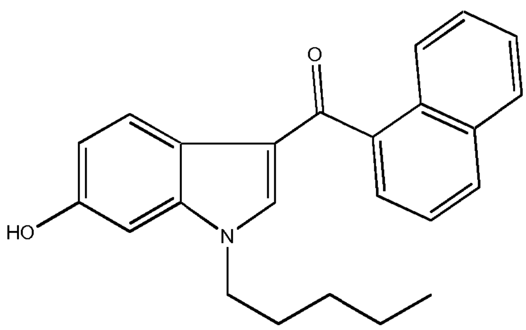

M5

**Figure 7**

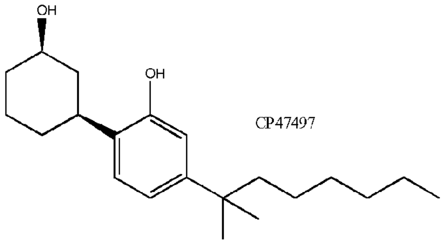

CP47497

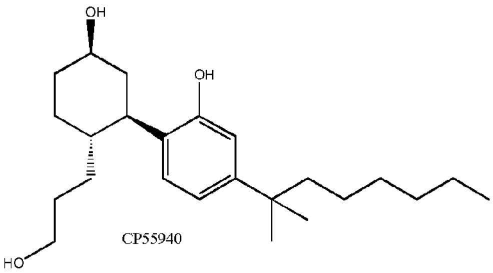

CP55940

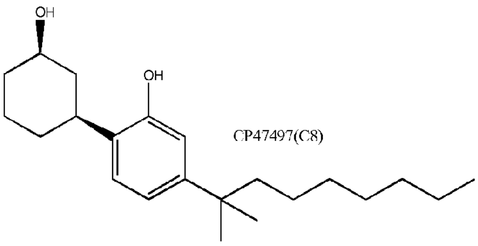

CP47497(C8)

**Figure 8**

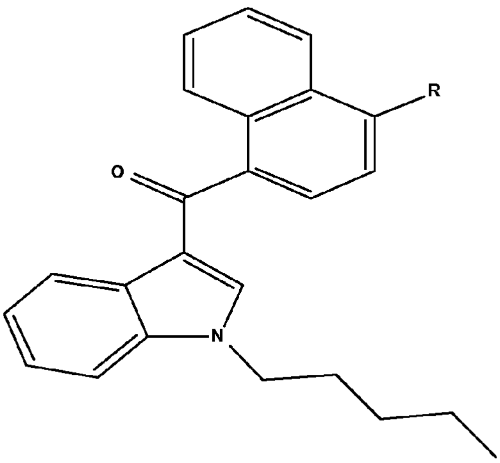

| | |
|---|---|
| R = H | JWH-018 |
| R = OMe | JWH-081 |
| R = Me | JWH-122 |
| R = Et | JWH-210 |
| R = Pr | JWH-182 |
| R = Cl | JWH-398 |

**Figure 9**: Chemical Structures of JWH-081, JWH-018, JWH-122, JWH-210, JWH-182 and JWH-398

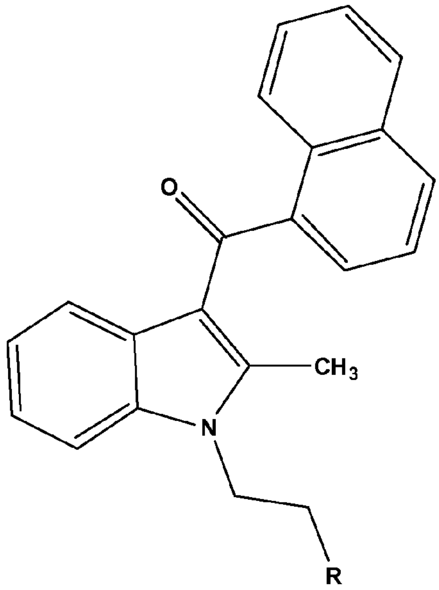

| | |
|---|---|
| **R = Methyl** | **JWH-015** |
| **R = Ethyl** | **JWH-016** |
| **R = Propyl** | **JWH-007** |

**Figure 10**: Chemical Structures of JWH-015, JWH-016 and JWH-007

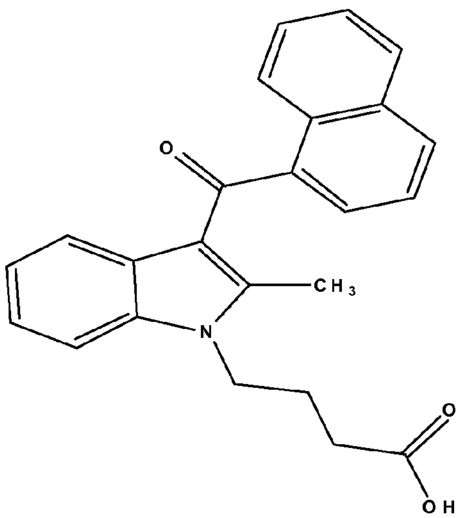

**Hapten-1**

**Figure 11**: Chemical Structure of Hapten-1

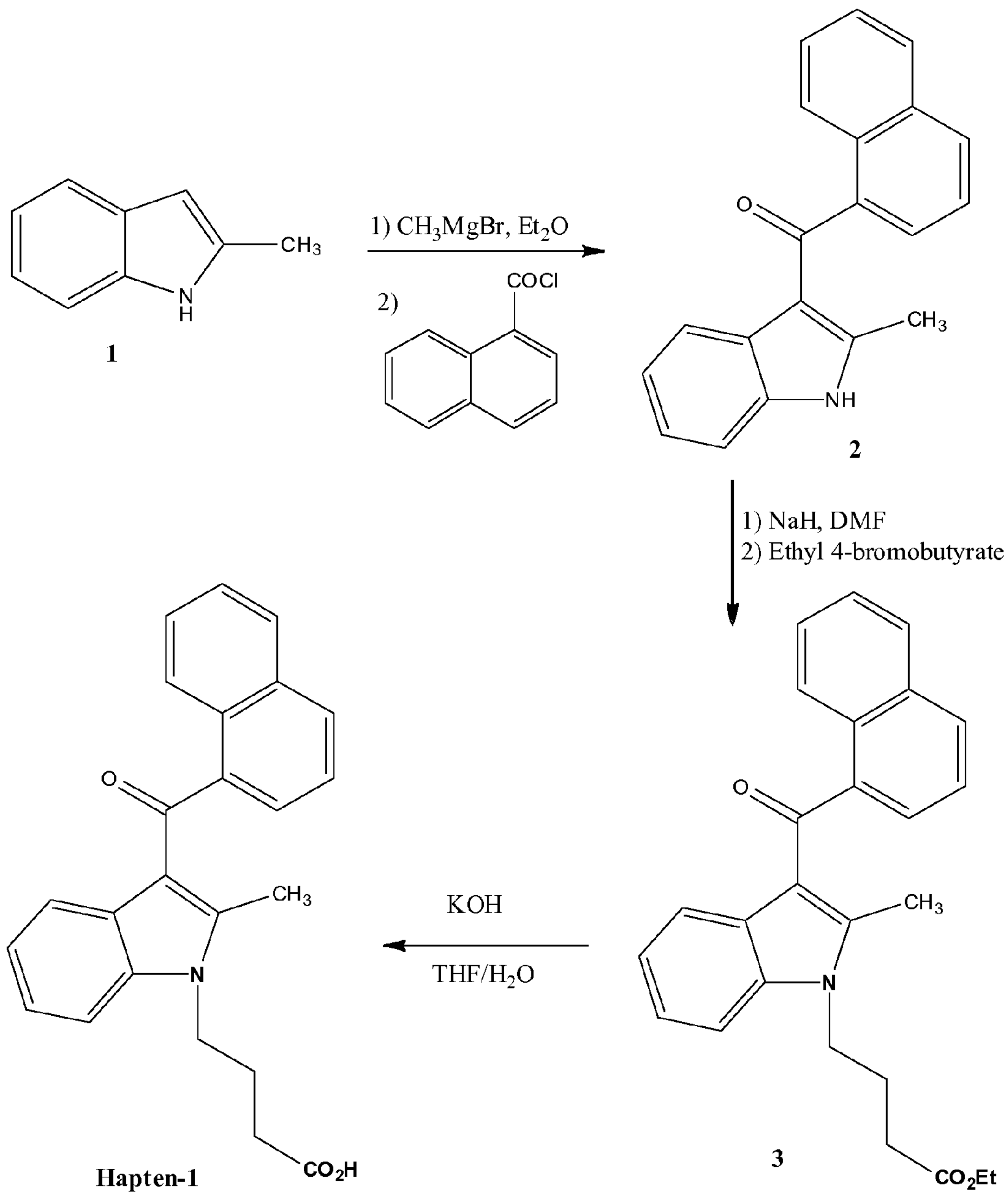


**Figure-12**: Chemical Reactions of the Synthesis of Hapten-1

Mal=maleimide
HRP= horseradish peroxidase

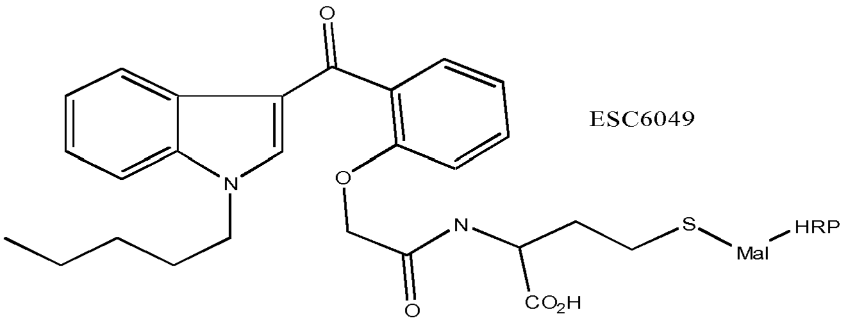

ESC6049

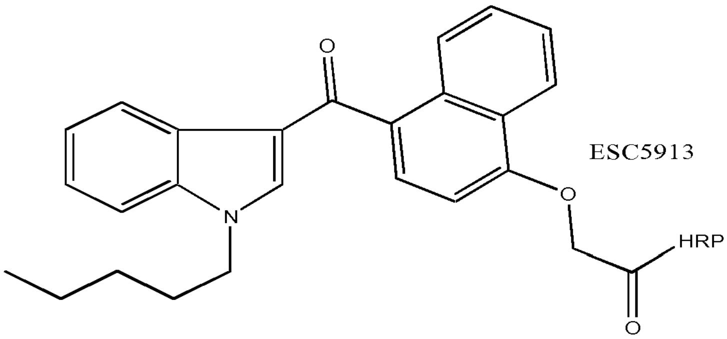

ESC5913

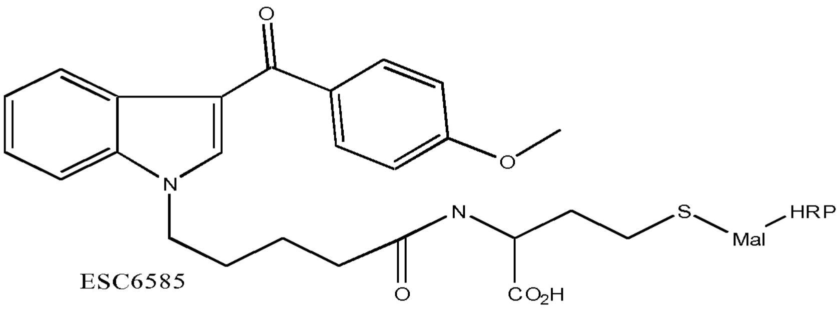

ESC6585

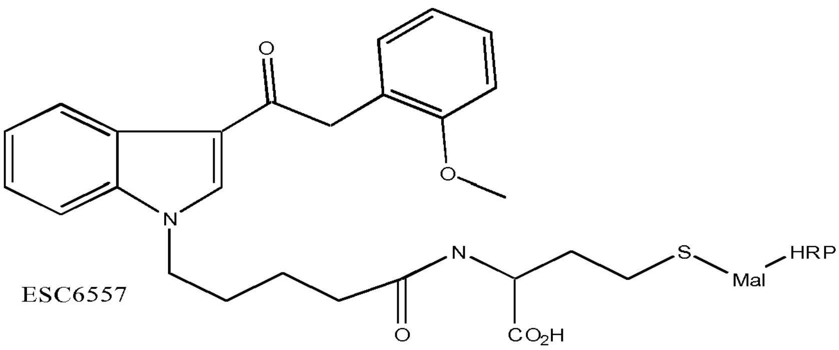

ESC6557

**Figure 13** – part 1

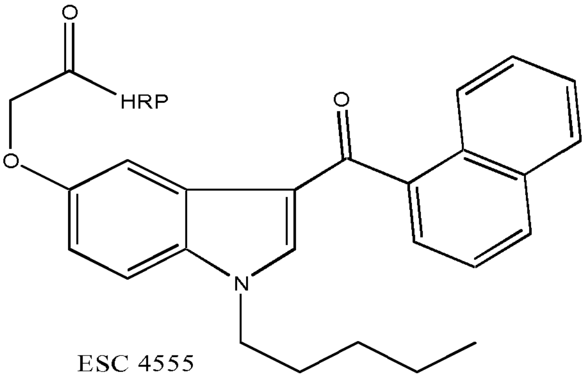

ESC 4555

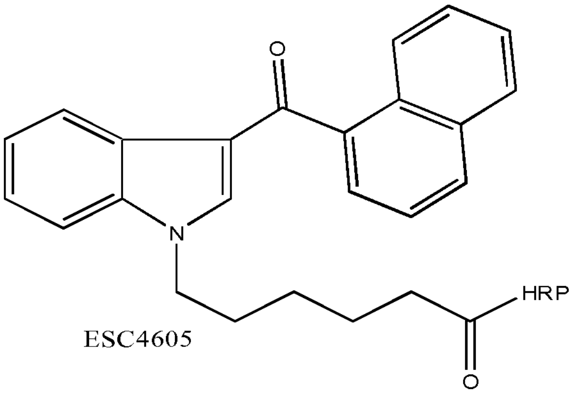

ESC4605

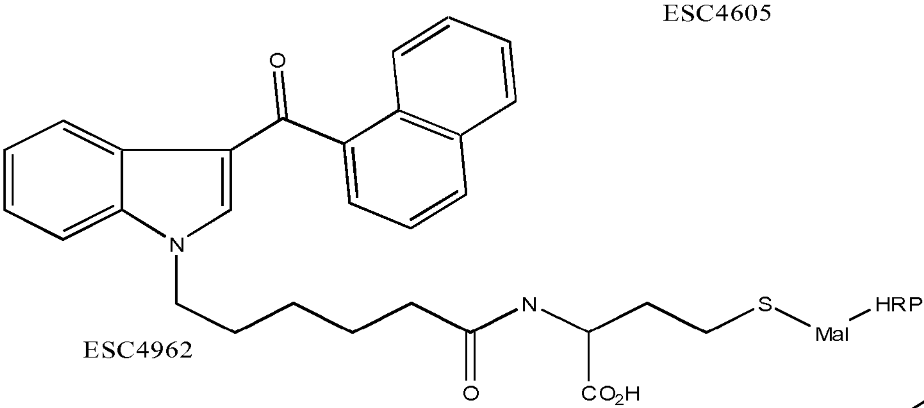

ESC4962

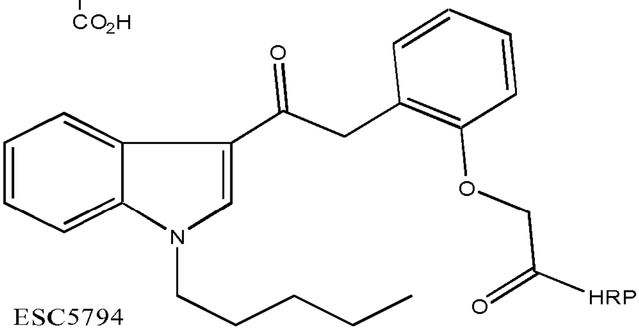

ESC5794

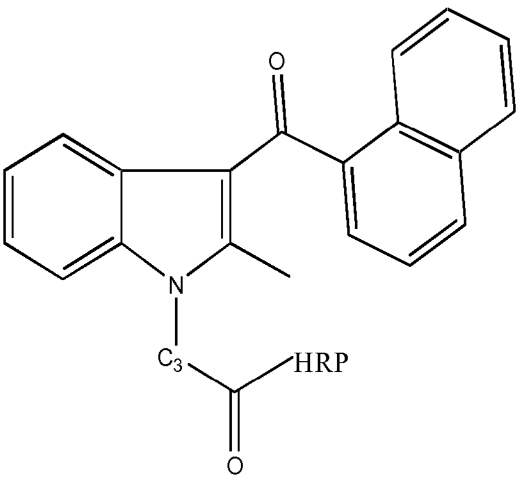

ESC6616

**Figure 13** – part 2

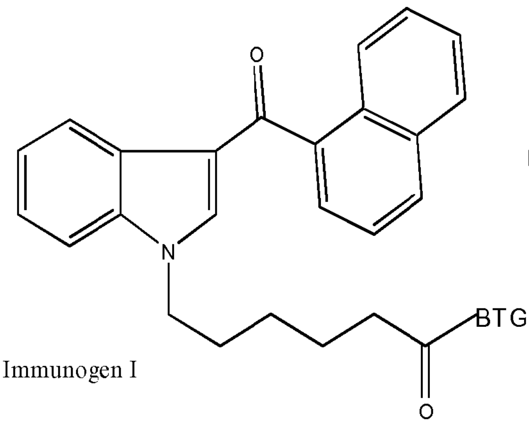

Immunogen I

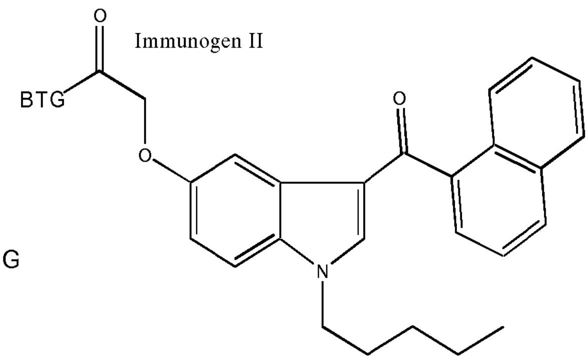

Immunogen II

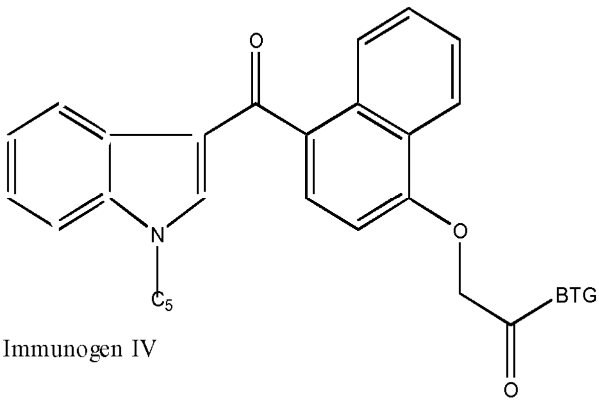

Immunogen IV

Immunogen VII

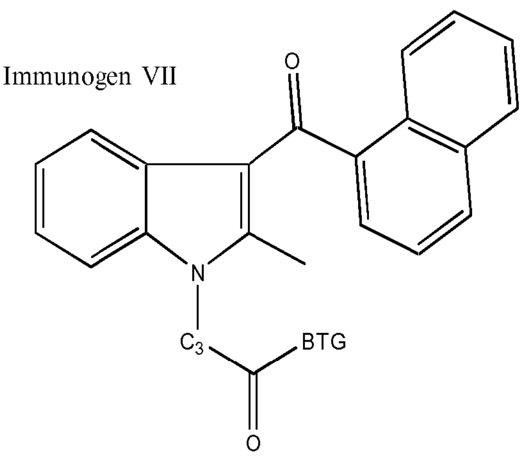

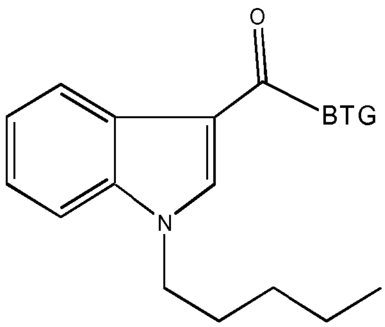

Immunogen VIII

**Figure 14**

Europäisches Patentamt
European Patent Office
Office européen des brevets

# EUROPEAN SEARCH REPORT

Application Number
EP 12 18 0490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 2 487 155 A1 (RANDOX LAB LTD [GB]) 15 August 2012 (2012-08-15) * e.g. claim 1-10; example 18,19; the whole document * ----- | 1-15 | INV. C07K16/44 G01N33/94 |
| X | Anonymous: "Randox Product List 2012", , 1 January 2012 (2012-01-01), XP55038674, Retrieved from the Internet: URL:http://www.randox.com/brochures/PDF Brochure/LT110.pdf [retrieved on 2012-09-19] | 1 | |
| Y | * page 13, line 14 from bottom of page; the whole document * ----- | 2-15 | |
| Y | WATANABE KAZUHITO ET AL: "CROSS-REACTIVITY OF VARIOUS TETRAHYDROCANNABINOL METABOLITES WITH A MONOCLONAL ANTIBODY AGAINST TETRAHYDROCANNABINOLIC ACID", JOURNAL OF HEALTH SCIENCE - EISEI KAGAKU, PHARMACEUTICAL SOCIETY OF JAPAN, JP, vol. 46, no. 4, 1 January 2000 (2000-01-01), pages 310-313, XP008070074, ISSN: 1344-9702 * e.g. abstract; the whole document * ----- | 2-15 | TECHNICAL FIELDS SEARCHED (IPC) C07K G01N |
| | -/-- | | |

The present search report has been drawn up for all claims

| Place of search | Date of completion of the search | Examiner |
|---|---|---|
| The Hague | 19 September 2012 | Gruber, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 18 0490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HIROYUKI TANAKA ET AL: "Monoclonal antibody against tetrahydrocannabinolic acid distinguishes Cannabis sativa samples from different plant species", FORENSIC SCIENCE INTERNATIONAL, vol. 106, no. 3, 1 December 1999 (1999-12-01), pages 135-146, XP55038622, ISSN: 0379-0738, DOI: 10.1016/S0379-0738(99)00193-0 * e.g. abstract; the whole document * ----- | 2-15 | |
| Y | SALAMONE SALVATORE J ET AL: "A NON-CANNABINOID IMMUNOGEN USED TO ELICIT ANTIBODIES WITH BROAD CROSS-REACTIVITY TO CANNABINOID METABOLITES", JOURNAL OF FORENSIC SCIENCES, CALLAGHAN AND CO, CHICAGO, IL, US, vol. 43, no. 4, 1 January 1998 (1998-01-01), pages 821-826, XP008070042, ISSN: 0022-1198 * e.g. abstract; the whole document * ----- | 2-15 | TECHNICAL FIELDS SEARCHED (IPC) |
| Y | H TANAKA: "Immunochemical Approach Using Monoclonal Antibody against delta9-Tetrahydrocannabinolic Acid (THCA) to Discern Cannabis Plants and to Investigate New Drug Candidates", CURRENT DRUG DISCOVERY TECHNOLOGIES, vol. 8, 1 January 2011 (2011-01-01), pages 3-15, XP55038676, * e.g. abstract; the whole document * ----- -/-- | 2-15 | |

The present search report has been drawn up for all claims

| Place of search | Date of completion of the search | Examiner |
|---|---|---|
| The Hague | 19 September 2012 | Gruber, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 18 0490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 0 736 529 A1 (HOFFMANN LA ROCHE [CH]) 9 October 1996 (1996-10-09)<br>* e.g. example 17;<br>the whole document *<br>----- | 2-15 | |
| Y | SEBASTIAN DRESEN ET AL: "Development and validation of a liquid chromatography-tandem mass spectrometry method for the quantitation of synthetic cannabinoids of the aminoalkylindole type and methanandamide in serum and its application to forensic samples", JOURNAL OF MASS SPECTROMETRY, vol. 46, no. 2, 1 February 2011 (2011-02-01), pages 163-171, XP55032002, ISSN: 1076-5174, DOI: 10.1002/jms.1877<br>* e.g. abstract;<br>the whole document *<br>----- | 2-15 | |

TECHNICAL FIELDS SEARCHED (IPC)

The present search report has been drawn up for all claims

| Place of search | Date of completion of the search | Examiner |
|---|---|---|
| The Hague | 19 September 2012 | Gruber, Andreas |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 18 0490

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2012

| Patent document cited in search report | Publication date | Patent family member(s) | Publication date |
|---|---|---|---|
| EP 2487155 A1 | 15-08-2012 | EP 2487155 A1 | 15-08-2012 |
| | | GB 245992 A | 21-01-1926 |
| | | US 2012208213 A1 | 16-08-2012 |
| EP 0736529 A1 | 09-10-1996 | AT 194339 T | 15-07-2000 |
| | | CA 2172663 A1 | 06-10-1996 |
| | | DE 69609108 D1 | 10-08-2000 |
| | | DE 69609108 T2 | 22-03-2001 |
| | | EP 0736529 A1 | 09-10-1996 |
| | | ES 2148617 T3 | 16-10-2000 |
| | | JP 4229990 B2 | 25-02-2009 |
| | | JP 8283258 A | 29-10-1996 |
| | | JP 2009051839 A | 12-03-2009 |
| | | US 5817766 A | 06-10-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1273349 A
- US 6900236 B


### Non-patent literature cited in the description


- **DRESEN et al.** *J. Mass Spectrom.,* 2010, vol. 45, 1186-1194
- **HUDSON et al.** *J. Anal. Toxicol.,* 2010, vol. 34, 252-260
- **MOLLER et al.** *Drug Test Anal.,* 24 September 2010
- **RANA et al.** Quantitative composition of synthetic cannabinomimetics in ''Herbal High'' products. *Poster at SOFT 2010 Annual Meeting, Richmond,* 2010
- **SOBOLEVSKY et al.** *Foren. Sci. Int.,* 2010, vol. 200 (1-3), 141-147
- **UCHIYAMA et al.** *Foren. Sci. Int.,* 2010, vol. 198, 31-38
- **WINTERMEYER et al.** *Anal. Bioanal. Chem.,* 2010, vol. 398, 2141-2153